(19) **Europäisches Patentamt**
**European Patent Office**
**Office européen des brevets**

(11) **EP 2 909 260 B1**

(12) # FASCICULE DE BREVET EUROPEEN

(45) Date de publication et mention
de la délivrance du brevet:
**31.08.2016 Bulletin 2016/35**

(21) Numéro de dépôt: **13789851.6**

(22) Date de dépôt: **15.10.2013**

(51) Int Cl.:
*C08J 9/28* (2006.01)          *C08J 9/30* (2006.01)
*B01F 17/00* (2006.01)

(86) Numéro de dépôt international:
**PCT/FR2013/052465**

(87) Numéro de publication internationale:
**WO 2014/060697 (24.04.2014 Gazette 2014/17)**

(54) **COMPOSITION COMPRENANT UNE PHASE INTERNE DISPERSÉE DANS UNE PHASE CONTINUE HYDROPHILE**

ZUSAMMENSETZUNG, DIE EINE INTERNE PHASE ENTHÄLT, DIE IN EINER KONTINUIERLICHEN HYDROPHILISCHEN PHASE DISPERGIERT IST

COMPOSITION COMPRISING AN INTERNAL PHASE DISPERSED IN A CONTINOUS HYDROPHILIC PHASE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.10.2012 FR 1259867**

(43) Date de publication de la demande:
**26.08.2015 Bulletin 2015/35**

(73) Titulaire: **Institut National de la Recherche Agronomique
(INRA)
75007 Paris (FR)**

(72) Inventeurs:
• **CAPRON, Isabelle**
**F-44300 Nantes (FR)**
• **BIZOT, Hervé**
**F-44240 Suce-sur-erdre (FR)**
• **CATHALA, Bernard**
**F-44240 LaChapelle Sur Erdre (FR)**

(74) Mandataire: **Jacobacci Coralis Harle
14-16 Rue Ballu
75009 Paris (FR)**

(56) Documents cités:
**WO-A1-2010/058148     WO-A1-2012/017160
US-A- 4 336 070     US-A1- 2010 261 803
US-B1- 6 365 642**

• **DATABASE WPI Week 200835 Thomson Scientific, London, GB; AN 2008-F01631 XP002692991, & CN 101 020 739 A (UNIV WUHAN SCI&ENG) 22 août 2007 (2007-08-22)**
• **DATABASE WPI Week 201242 Thomson Scientific, London, GB; AN 2012-E15526 XP002692992, & CN 102 391 416 A (UNIV EAST CHINA SCI&TECHNOLOGY) 28 mars 2012 (2012-03-28)**
• **TZOUMAKI M.V., MOSCHAKIS T., KIESSEOGLOU V., BILIADERIS C.G.: "Oil-in-water emulsions stabilized by chitin nanocrystal particles", FOOD HYDROCOLLOIDS, vol. 25, 1 août 2011 (2011-08-01), pages 1521-1529, XP002692993,**
• **LI C; SUN P; YANG C: "Emulsion stabilized by starch nanocrystals", STARCH/STÄRKE, vol. 64, 1 juin 2012 (2012-06-01), pages 497-502, XP002692994,**
• **TAN Y; XU K; LIU C; LI Y; LU C; WANG P: "Fabrication of starch-based nanospheres to stabilize pickering emulsion", CARBOHYDRATE POLYMERS, vol. 88, 16 février 2012 (2012-02-16), pages 1358-1363, XP002692995,**
• **GUREVITCH I; SILVERSTEIN M S: "Polymerized pickering HIPEs: Effects of synthesis parameters on porous structure", JOURNAL OF POLYMER SCIENCE, PART B: POLYMER PHYSICS, vol. 48, 1 avril 2010 (2010-04-01), pages 1516-1525, XP002692996,**

- **KALASHNIKOVA I., BIZOT H., CATHALA B., CAPRON I.: "New Pickering Emulsions stabilized by bacterial cellulose nanocrystals.", LANGMUIR, vol. 27, 23 mai 2011 (2011-05-23), pages 7471-7479, XP002692997,**
- **MENNER A ET AL: "Tough reinforced open porous polymer foams via concentrated emulsion templating", POLYMER, ELSEVIER SCIENCE PUBLISHERS B.V, GB, vol. 47, no. 22, 18 octobre 2006 (2006-10-18), pages 7628-7635, XP028061139, ISSN: 0032-3861, DOI: 10.1016/J.POLYMER.2006.09.022 [extrait le 2006-10-18]**

**Description**

## DOMAINE DE L'INVENTION

La présente invention se rapporte au domaine des compositions à phase interne élevée, avantageusement une émulsion ou une mousse, de préférence une émulsion dite à « moyenne phase interne » ou « haute phase interne ».

## ART ANTERIEUR

Certaines compositions comprennent une phase interne dispersée dans une phase continue, à savoir en particulier les émulsions et les mousses.

Une émulsion est un mélange, macroscopiquement homogène mais microscopiquement hétérogène, de deux substances liquides non miscibles.

Les deux substances liquides en présence sont appelées des phases. Une phase est continue ; l'autre phase discontinue interne est dispersée dans la première phase sous forme de gouttelettes.

Une mousse est très similaire à une émulsion : le gaz (souvent de l'air) est dispersé en nombreuses bulles (on parle de « phase dispersée » ou de « phase interne »), tandis que le liquide aqueux est entièrement continu (on parle de « phase continue »).

L'obtention d'émulsions du type huile-dans-eau, stabilisées par des nanocristaux de chitine, est connue par exemple de TZOUMAKI et al., « Oil-in-water emulsions stabilized by chitin nanocrystal particles », FOOD HYDROCOLLOIDS, 25 (2011), pages 1521-1529.

Dans ce document, les émulsions obtenues comportent un volume de phase hydrophobe qui est inférieur à 50%.

D'autres compositions consistent en des systèmes dispersés immiscibles dans lesquels la phase interne, encore appelée phase dispersée, occupe un volume supérieur à environ 50 pour cent du volume total de la composition.

Les émulsions à phase interne élevée consistent classiquement en des émulsions dites à « moyenne phase interne » ou « haute phase interne », encore appelées émulsions de type « MIPE » (pour « Medium Internal Phase Emulsions ») ou « HIPE » (pour « High Internai Phase Emulsions ») respectivement.

Les émulsions à haute phase interne ou HIPE consistent en des systèmes dispersés immiscibles liquide/liquide dans lesquels la phase interne occupe un volume supérieur à environ 74% - 75% du volume total de l'émulsion, c'est-à-dire un volume supérieur à ce qui est possible géométriquement pour l'empaquetage compact de sphères monodisperses.

Les émulsions à moyenne phase interne ou MIPE consistent quant à elles en des systèmes dispersés immiscibles liquide/liquide dans lesquels la phase interne occupe un volume compris entre environ 50% et 74 - 75 % du volume total de l'émulsion.

L'obtention d'émulsions du type eau-dans-huile à phase interne élevé (du type MIPE ou HIPE), et leur utilisation pour la fabrication de mousses polymères, est par exemple décrite dans une demande PCT n°WO-2010/058148.

Dans cette demande PCT n°WO-2010/058148, l'émulsion à haute phase interne est stabilisée par des particules dérivées de la cellulose ou de la chitine, qui sont pour cela fonctionnalisées en surface par hydrophobisation.

Or, l'emploi de telles particules transformées est susceptible de poser des problèmes de sécurité, en particulier pour les applications alimentaires, avec les questions de leur innocuité pour le consommateur.

L'obtention de telles particules transformées oblige une étape supplémentaire de modification en surface.

Ces particules transformées ne sont en plus pas adaptées pour l'encapsulation de constituants hydrophobes, du fait qu'elles sont prévues pour des émulsions du type eau-dans-huile.

Compte tenu de ce qui précède, il existe un besoin pour de nouvelles compositions d'émulsion à phase interne élevée du type huile-dans-l'eau, pour diverses applications industrielles, en particulier alimentaire.

Plus généralement, il existe un besoin pour des compositions, en particulier des émulsions, à phase interne élevée qui soient stabilisées par des agents disponibles en grande quantité, biodégradables, non-toxiques, renouvelables, peu chères, de très faibles densités et éventuellement facilement adaptables par modification de surface.

## RESUME DE L'INVENTION

La présente invention concerne une composition (avantageusement une émulsion ou une mousse) comprenant une phase interne, notamment hydrophobe ou gazeuse, dispersée dans une phase continue hydrophile, possédant un pourcentage de phase interne supérieur à 50%.

Cette composition est caractérisée par le fait qu'elle contient des nanocristaux d'un polysaccharide autre que la cellulose, localisés à l'interface entre ladite phase interne et ladite phase continue.

La composition selon l'invention contient ainsi avantageusement :

(i) des nanocristaux issus d'un unique polysaccharide ou d'au moins deux polysaccharides différents, à l'exclusion des nanocristaux de cellulose, ou

(ii) des nanocristaux issus d'un unique polysaccharide ou d'au moins deux polysaccharides différents, autres que la cellulose, en combinaison avec des nanocristaux issus de la cellulose.

Les nanocristaux de polysaccharide, autres que les nanocristaux de cellulose, sont avantageusement choisis parmi les nanocristaux qui sont chargés positivement, à savoir de préférence les nanocristaux de chitine.

Ces nanocristaux de polysaccharide sont choisis de préférence parmi :

(i) exclusivement des nanocristaux de polysaccharide chargés positivement, à l'exclusion de nanocristaux de cellulose, ou

(ii) des nanocristaux de polysaccharide chargés positivement, autre que la cellulose, mélangés avec des nanocristaux issus d'au moins un polysaccharide chargés négativement.

Une telle composition consiste avantageusement en une émulsion ou en une mousse.

La composition consiste avantageusement en une composition d'émulsion du type à haute phase interne ou HIPE, possédant un pourcentage de phase interne supérieur à 75%.

De manière alternative, la composition d'émulsion est du type à moyenne phase interne ou MIPE, possédant un pourcentage de phase interne compris entre 50% eut 75%.

La présente invention concerne encore un procédé d'obtention d'une composition comprenant une phase interne, notamment hydrophobe ou gazeuse, dispersée dans une phase continue hydrophile, possédant un pourcentage de phase interne supérieur à 50%.

Ce procédé est caractérisé en ce qu'il comprend les opérations suivantes :

a) une étape d'incorporation de nanocristaux d'un polysaccharide autre que la cellulose, dans une phase hydrophile,

b) une opération de fourniture de ladite phase hydrophile contenant les nanocristaux de polysaccharide et d'une phase destinée à constituer la phase interne, en particulier une phase interne hydrophobe,

c) une opération de formation de ladite composition par dispersion de ladite phase interne dans ladite phase hydrophile.

Les nanocristaux de polysaccharide incorporés à l'étape a) sont avantageusement choisis parmi les nanocristaux qui sont chargés positivement, à savoir de préférence les nanocristaux de chitine.

Ces nanocristaux de polysaccharide sont choisis en particulier parmi :

(i) exclusivement des nanocristaux de polysaccharide chargés positivement, à l'exclusion de nanocristaux de cellulose, ou

(ii) des nanocristaux de polysaccharide chargés positivement, autre que la cellulose, mélangés avec des nanocristaux issus d'au moins un polysaccharide chargés négativement.

A l'issue de l'étape c), la composition obtenue consiste avantageusement en une émulsion ou en une mousse.

Selon un premier mode de réalisation, l'opération c) de formation d'une émulsion consiste en une opération « séquentielle » qui comprend :

c.1) une étape d'obtention d'une émulsion huile-dans-l'eau, intermédiaire, ayant un rapport volumique phase interne hydrophobe / phase continue hydrophile d'au moins 5/95,

c.2) une étape d'obtention de la composition émulsion possédant un pourcentage de phase interne supérieur à 50%, comprenant :

c.2.1) une étape d'ajout d'un volume de phase hydrophobe à l'émulsion intermédiaire obtenue à l'étape c.1), et agitation du mélange ainsi obtenu, et/ou

c.2.2) une étape de concentration de l'émulsion intermédiaire obtenue à l'étape c.1), par retrait d'au moins une partie de ladite phase hydrophile.

Dans ce cas, à l'étape c.1), l'émulsion huile-dans-eau intermédiaire a avantageusement un pourcentage de phase interne inférieur ou égal à 50%.

Selon un second mode de réalisation, l'opération c) de formation d'une émulsion consiste avantageusement en une opération « directe », qui consiste à mélanger la phase hydrophile contenant les nanocristaux de polysaccharide et la phase hydrophobe, pour l'obtention directement de ladite émulsion possédant un pourcentage de phase interne supérieur à 50%.

De manière générale, à l'issue de l'étape c), la composition d'émulsion formée est (i) du type à haute phase interne ou HIPE, possédant un pourcentage de phase interne supérieur à 75% ou (ii) du type à moyenne phase interne ou MIPE, possédant un pourcentage de phase interne compris entre 50% et 75%.

## DESCRIPTION DES FIGURES

Fig. 1 : Distribution du diamètre des gouttes ($\mu$m) en fonction de la concentration en nanocristaux de chitine dans la phase aqueuse (g/L) ;

Fig. 2 : Distribution du diamètre des gouttes ($\mu$m) en fonction du pH du milieu, ajusté par la modification de la concentration en HCl (nanocristaux de chitine à 3g/L, NaCl 20mM) ;

Fig. 3 : Distribution des diamètres des gouttes ($\mu$m), après stockage à différentes températures (nanocristaux de chitine à 3g/L, HCl 0,01 mM, NaCl 20mM) ;

Fig. 4 : Distribution des diamètres des gouttes ($\mu$m), avant et après séchage (nanocristaux de chitine à 5g/L, HCl 0,01 mM, NaCl 50mM) ;

Fig. 5 : Evolution du pourcentage de phase interne pour une suspension mixte nanocristaux de chitine 1,5g/L / nanocristaux de cellulose 1,5g/L (« ChitineNC 1,5g/L + CelluloseNC 1,5g/L »), en comparaison d'une suspension témoin de nanocristaux de chitine à 3g/L (« Témoin ChitineNC 3g/L »), en fonction du volume d'huile ajouté en mL

## DESCRIPTION DETAILLEE DE L'INVENTION

La présente invention fournit une nouvelle composition à phase interne élevée, c'est-à-dire avantageusement une composition d'émulsion à moyenne phase interne (MIPE) ou à haute phase interne (HIPE), ainsi que des procédés pour l'obtention d'une telle composition.

De manière générale, la demanderesse a montré que, de manière inattendue, une composition d'émulsion du type MIPE/HIPE peut être stabilisée par des nanocristaux d'un polysaccharide autre que la cellulose.

La demanderesse a également montré que, de manière inattendue, une composition sous forme de mousse peut être stabilisée par des nanocristaux d'un polysaccharide autre que la cellulose.

Les nanocristaux de polysaccharide, autres que les nanocristaux de cellulose, présentent avantageusement les avantages suivants :

- une adsorption irréversible engendrant une grande stabilité des émulsions MIPE/HIPE (l'énergie de détachement nécessaire pour la désorption des nanocristaux de l'interface est telle qu'il est considéré que cette désorption ne s'effectuera pas spontanément),
- non toxicité,
- biodégradables,
- issu d'une source biologique, et
- modifiable facilement par post-traitement.

Ces nanocristaux de polysaccharide, autres que ceux de cellulose, possèdent avantageusement une structure du type bâtonnet anisotrope, assurant des pourcentages de recouvrement et de porosité variables.

De préférence encore, les nanocristaux de polysaccharide, autres que ceux de cellulose, sont avantageusement choisis parmi les nanocristaux issus de polysaccharides comportant des monomères de glucose, ou dérivé de glucose (par exemple de glucosamine ou de dérivé de glucosamine), reliés par des liaisons béta 1,4.

Les nanocristaux de polysaccharide choisis possèdent avantageusement une charge positive modulable (alors que la plupart des biopolymères sont chargés négativement), autorisant des combinaisons originales et multiples.

### Composition selon l'invention

La composition selon l'invention comprend ainsi une phase interne dispersée dans une phase continue hydrophile, possédant un pourcentage de phase interne supérieur à 50%.

Cette composition contient des nanocristaux d'un polysaccharide autre que la cellulose ; ces nanocristaux sont localisés à l'interface entre la phase interne et la phase continue hydrophile.

Une telle composition consiste avantageusement en :

- une composition d'émulsion du type à moyenne phase interne (MIPE) ou à haute phase interne (HIPE), dans laquelle ladite phase interne est constituée par une phase liquide hydrophobe, ou

- une composition sous forme de mousse, dans laquelle ladite phase interne est constituée par une phase gazeuse.

*Définition générale*

La composition selon l'invention consiste avantageusement en une émulsion ou en une mousse.

Par « émulsion » ou « composition d'émulsion », on entend un mélange, macroscopiquement homogène mais microscopiquement hétérogène, de deux phases liquides non miscibles.

Dans la présente description, les notions de « émulsion » ou de « composition d'émulsion » sont employées indifféremment.

Dans une émulsion du genre « huile-dans-l'eau », au sens de l'invention, (i) la phase continue dispersante hydrophile consiste en une phase aqueuse et (ii) la phase interne dispersée est une phase hydrophobe.

Une émulsion huile-dans-l'eau peut être aussi désignée par le sigle « H/E » dans la présente description.

Par « mousse », on entend un milieu complexe constitué d'un matériau solide ou liquide intimement mêlé à du gaz.

Selon l'invention, la mousse consiste avantageusement en une mousse liquide aqueuse dans laquelle (i) la phase continue dispersante hydrophile consiste en une phase aqueuse et (ii) la phase interne dispersée est une phase gazeuse.

Cette mousse peut consister en une mousse dite « sèche », c'est-à-dire une mousse peu hydratée.

Plus précisément, une mousse est dite « sèche » lorsque le liquide n'occupe qu'une partie négligeable du volume total de la mousse ; par convention, on suppose ainsi que la fraction volumique de liquide est alors nulle.

Expérimentalement, la fraction volumique de liquide peut descendre à $10^{-2}$, voire $10^{-4}$.

La fraction volumique de liquide correspond au rapport entre le volume de liquide et le volume total de mousse ($V_{liquide}$ / $V_{mousse}$).

Cette valeur est reliée à la densité ρ de la mousse, par la formule suivante :

$$\rho = \rho_l \phi_l + \rho_g (1-\phi_l)$$

avec

$\rho_l$ et $\rho_g$, les densités respectives de la solution et du gaz,

$\phi_l$ correspondant à la fraction volumique de liquide,

$\phi_l{}^*$ correspondant à la fraction volumique de liquide, critique, dont les gouttes sont non déformées (cette notion est encore définie dans « Les mousses : structure et dynamique », L. Cantat et al. Paris. ; Ed Belin, 2010 - 278 pages - ISBN : 978-2-7011-4284-5).

On définit ainsi 3 types de structures de mousse :

- si $\phi_l{}^* < \phi_l$: les bulles sont sphériques et sans contact, correspondant à un liquide bulleux,
- si $0,05 < \phi_l < \phi_l{}^*$: les bulles se touchent et prennent la forme de sphères écrasées, correspondant à une mousse humide,
- si $\phi_l < 0,05$ : les bulles se touchent et prennent la forme de polyèdres, correspondant à une mousse sèche.

En d'autres termes, le pourcentage de phase interne est compris entre 64% et 95 %, environ, pour une mousse humide ; la fraction volumique en phase interne est au-delà de 95% pour une mousse sèche.

Les aspects techniques relatifs à ces mousses sont encore décrits dans le document suivant : « Les mousses : Structure et Dynamique », L. Cantat et al ; Paris ; Ed Belin, 2010 - 278 pages - ISBN : 978-2-7011-4284-5.

Dans la présente description les termes « phase huileuse » et « phase hydrophobe », peuvent être indifféremment utilisés pour désigner le liquide huileux utilisé pour la préparation d'une émulsion du genre huile-dans-l'eau.

La composition selon l'invention comporte au moins une phase interne.

Dans la présente description, à l'exception des aspects spécifiques aux émulsions, les termes « phase interne », « phase interne hydrophobe », « phase dispersée », « phase dispersée hydrophobe » peuvent être indifféremment utilisés pour désigner :

- la phase huileuse dispersée d'une émulsion du genre huile-dans-l'eau, ou
- le gaz dispersé dans une mousse liquide aqueuse.

Dans la présente description, les termes « phase aqueuse » et « phase hydrophile » peuvent être indifféremment utilisés pour désigner le liquide aqueux utilisé pour la préparation d'une émulsion du genre huile-dans-l'eau ou d'une mousse.

Dans la présente description, les termes « phase continue », « phase continue hydrophile » et « phase continue aqueuse » peuvent être indifféremment utilisés pour désigner la phase aqueuse dispersante d'une émulsion huile-dans-l'eau ou d'une mousse.

Par « pourcentage de phase interne » d'une composition d'émulsion ou d'une mousse, on entend selon l'invention le rapport, exprimé en pourcentage volumique, entre (i) le volume de phase interne dispersée dans la phase continue hydrophile et (ii) le volume total de la composition résultante.

Pour une mousse, la notion de « pourcentage de phase interne » correspond avantageusement, de manière équivalente, à la notion de « fraction volumique de la phase dispersée ».

Une composition à « phase interne élevée » est ainsi un système dispersé immiscible liquide/liquide ou liquide/gaz dans lequel la phase interne, encore appelée phase dispersée, occupe un volume supérieur à environ 50 %, de préférence à environ 55 %, du volume total de la composition.

Une « émulsion à phase interne élevée » est ainsi un système dispersé immiscible liquide/liquide dans lequel la phase interne, encore appelée phase dispersée, occupe un volume supérieur à environ 50 %, de préférence à environ 55 %, du volume total de l'émulsion.

On rappelle qu'une émulsion à haute phase interne (HIPE) consiste en un système dispersé immiscible liquide/liquide dans lequel la phase interne, encore appelée phase dispersée, occupe un volume supérieur à environ 74-75 pour cent du volume total de l'émulsion, c'est-à-dire un volume supérieur à ce qui est possible géométriquement pour l'empaquetage compact de sphères monodisperses, c'est à dire d'une population de sphères de taille homogène.

On rappelle encore qu'une émulsion à moyenne phase interne (MIPE) consiste en un système dispersé immiscible liquide/liquide dans lequel la phase interne occupe un volume compris entre 50 et 74-75 pour cent du volume total de l'émulsion.

Selon l'invention, l'émulsion à moyenne phase interne (MIPE) consiste avantageusement en un système dispersé immiscible liquide/liquide dans lequel la phase interne occupe un volume compris entre 50, de préférence 55, et 74-75 pour cent du volume total de l'émulsion.

Dans certaines situations, lorsqu'une émulsion de type HIPE est préparée conformément au procédé défini ci-dessus, on peut obtenir une phase d'émulsion comprenant la phase hydrophobe qui est dispersée dans la phase continue hydrophile sous forme d'émulsion, avec éventuellement (i) une phase huileuse constituée d'un volume de la phase hydrophobe qui est présente dans la composition sous une forme non dispersée (ce volume étant mesuré) et/ou (ii) une phase aqueuse (ne faisant pas partie de l'émulsion).

Le pourcentage de phase interne est calculé par (i) mesure du volume de la phase hydrophobe non dispersée, laquelle en général surmonte la phase d'émulsion, (ii) mesure du volume de la phase d'émulsion, puis (iii) calcul du volume de phase hydrophobe qui est sous forme dispersée dans la phase d'émulsion, étant entendu que le volume total de phase hydrophobe contenu dans la composition est connu.

Par « rapport volumique phase interne hydrophobe / phase continue hydrophile », notamment pour une émulsion de type MIPE ou HIPE, on entend selon l'invention le rapport entre (i) le volume de la phase hydrophobe intégré dans l'émulsion, et (ii) le volume de la phase hydrophile intégré dans l'émulsion.

Ce dernier rapport est uniquement indicatif, en ce sens qu'il est également fonction de la quantité de nanocristaux intégrée dans l'émulsion. D'une manière générale, les essais ont été réalisés avec une phase hydrophile contenant des nanoparticules en suspension à une concentration de 0,3 à 0,8 g/L. Cette concentration n'est aucunement limitative ; la limite la plus fiable est avantageusement, sans n'être aucunement limitée, un taux de recouvrement de 80% lors de la fabrication de l'émulsion de Pickering. Dans le cas d'un procédé de fabrication « séquentiel » développé ci-après, si la condition de stabilité de l'émulsion de Pickering est remplie (étape c.1)), le procédé peut être poursuivi par l'ajout de la phase hydrophobe ou par le retrait d'au moins une partie de la phase hydrophile pour former l'émulsion MIPE ou HIPE (étape c.2)).

*Pourcentage de phase interne*

Selon l'invention, une composition comportant une « phase interne élevée » possède un pourcentage de phase interne supérieur à 50%, de préférence supérieur à 55%.

Selon un mode de réalisation, l'émulsion formée est avantageusement du type à moyenne phase interne (MIPE), possédant un pourcentage de phase interne compris entre 50% et 75%, de préférence entre 55% et 75%, avantageusement encore entre 60% et 75%, avantageusement encore entre 65% et 75% et avantageusement encore entre 70% et 75%.

Selon un autre mode de réalisation, l'émulsion formée peut encore être du type à haute phase interne (HIPE), possédant un pourcentage de phase interne supérieur à 75%, de préférence encore supérieur à 80%, de préférence encore supérieur à 85% voire encore supérieur à 90%. Ce pourcentage de phase interne est encore avantageusement compris entre 80% et 90%, de préférence entre 85% et 90%.

Selon encore un autre mode de réalisation, la composition se présente sous la forme d'une mousse dont le pourcentage de phase interne, correspondant à la fraction volumique de phase dispersée, est supérieur à 50%, de préférence supérieur à 55%, de préférence encore entre 64% et 95%.

Dans le cas d'une mousse dite « sèche », le pourcentage de phase interne est avantageusement supérieur à 95%.

*Rapport volumique*

Dans certains modes de réalisation, on forme une composition d'émulsion MIPE ou HIPE ayant un rapport volumique phase interne hydrophobe / phase continue hydrophile supérieur à 60/40, de préférence encore supérieur à 80/20.

Par « supérieur à 60/40 » ou « d'au moins 60/40 », on entend une phase interne hydrophobe dont la valeur est avantageusement supérieure à 60 dans le rapport volumique, à savoir notamment 65/35, 70/30, 75/25, 80/20, 85/15 ou 90/10.

Par « supérieur à 80/20 » ou « d'au moins 80/20 », on entend une phase interne hydrophobe dont la valeur est avantageusement supérieure à 80 dans le rapport volumique, à savoir notamment 85/15 ou 90/10.

*Nanocristaux d'un polysaccharide autre que la cellulose*

La composition, en particulier une composition d'émulsion, est ainsi stabilisée par des nanocristaux de polysaccharide qui consistent, pour certains au moins, en des nanocristaux autres que des nanocristaux de cellulose.

Les polysaccharides (parfois appelés glycanes, polyosides, polyholosides ou glucides complexes) sont des polymères constitués de plusieurs oses (encore appelés sucres ou « anhydroglucose unit » ou « AGU ») liés entre eux.

Par « polysaccharides », on entend en particulier les polymères formés par la condensation répétitive d'oses par liaison osidique, avantageusement d'au moins 10 unités osidiques, pour atteindre plusieurs centaines ou milliers d'unités.

Les nanocristaux de polysaccharide sont connus de l'art antérieur, souvent sous la dénomination de « whiskers » ou de « nanowiskers ».

L'expression « nanocristaux » signifie qu'il s'agit de cristaux dont au moins une dimension est de taille nanoscopique ou submicronique, constitués de chaînes macromoléculaires orientées dans le sens de la fibre de façon parallèle ou antiparallèle.

De tels nanocristaux de polysaccharide peuvent être issus de diverses sources, par exemple d'origine végétale, bactérienne, animale, fongique ou amibienne ; ils peuvent être obtenus par dégradation de la source ou par recristallisation.

La composition selon l'invention peut contenir notamment :

(i) des nanocristaux issus d'un polysaccharide ou d'un mélange de polysaccharides, autres que la cellulose, ou

(ii) des nanocristaux issus d'un polysaccharide ou d'un mélange de polysaccharides, autres que la cellulose, mélangés avec des nanocristaux issus de la cellulose.

Par « nanocristaux de cellulose », on entend des nanocristaux connus de l'art antérieur, souvent sous la dénomination de « whiskers » de cellulose, de « nanowiskers » de cellulose ou de « nanocellulose ».

De tels nanocristaux de cellulose peuvent être issus de diverses sources : végétale (par exemple pulpe de bois, coton ou algues), animale (par exemple tunicier), bactérienne, cellulose régénérée ou cellulose mercerisée. Ils sont par exemple décrits dans le document Samir et al. (2005, Biomacromolecules, Vol.6 : 612-626) ou dans le document Elazzouzi-Hafraoui et al. (Biomacromolecules. 2008;9(1):57-65.).

Les nanocristaux de polysaccharide, autres que ceux de cellulose, sont avantageusement choisis parmi les nanocristaux issus de polysaccharides dits « de structure », c'est-à-dire des polymères de glucose ou d'un dérivé de glucose qui ne sont pas ramifiés et dont la liaison entre unité est une liaison anomérique de type $\beta$.

A cet égard, les nanocristaux de polysaccharide, autres que ceux de cellulose, sont avantageusement choisis parmi les nanocristaux issus des polysaccharides suivants :

- de glucanes, polymères de D-glucose,
- de galactanes, polymères de D-galactose,
- de xylanes, polymères de D-xylose, et
- de polymères de D-glucosamine, comprenant notamment la chitine.

Par « glucane », on entend la famille des α-glucanes, y compris les polysaccharides suivants :

- l'amidon, α-1,4- et α-1,6-glucane
- l'amylopectine, α-1,4- et α-1,6-glucane
- l'amylose, α-1,4-glucane.

Par « glucane », on entend également la famille des β-glucanes, y compris les polysaccharides suivants :

- le curdlan, β-1,3-glucane
- le laminarin, β-1,3- et β-1,6-glucane
- le lentinane, pur β-1,6:β-1,3-glucane
- le pamylon
- le pleurane, β-1,3- et β-1,6-glucane
- le zymosan, β-1,3-glucane

De préférence encore, ces nanocristaux de polysaccharide, autres que ceux de cellulose, sont avantageusement choisis parmi les nanocristaux issus de polysaccharides comportant des monomères de glucose, ou dérivé de glucose (par exemple de glucosamine ou de dérivé de glucosamine notamment du type N-acétyl-D-glucosamine), reliés par des liaisons béta 1,4.

Parmi les nanocristaux de polysaccharide, il est choisi en particulier ceux comportant une charge positive (alors que la plupart des biopolymères sont chargés négativement), autorisant des combinaisons originales et multiples.

Cette charge positive est avantageusement modulable.

Cette charge positive peut être intrinsèque aux nanocristaux d'intérêt (particules non fonctionnalisées) ou peut être obtenue par fonctionnalisation (avantageusement par greffage d'une fonction N-acétylamine).

La composition selon l'invention peut ainsi contenir notamment :

(i) exclusivement des nanocristaux de polysaccharide chargés positivement, à l'exclusion de nanocristaux de cellulose, ou

(ii) des nanocristaux de polysaccharide chargés positivement, autre que la cellulose, mélangés avec des nanocristaux issus d'au moins un polysaccharide avantageusement chargés négativement.

A cet égard, les nanocristaux de polysaccharide sont ainsi avantageusement choisis parmi les nanocristaux de chitine, désignés encore généralement en anglais sous le nom de « chitin nano-crystal » ou « ChN ».

Le nom chimique de la molécule de chitine est le poly N-acétyl-D-glucosamine, β-(1,4)-2-acétamido-2-désoxy-D-glucose ou plus simplement [N-acétyl-D-glucosamine β-(1,4) N-acétyl-D-glucosamine]$_n$.

La chitine englobe avantageusement les polysaccharides composés d'unités de N-acétyl-β-D-glucosamine (de 50 à 100%) et d'unités D-glucosamine (de 0 à 50%).

Dans le cadre de l'invention, on nommera avantageusement « nanocristaux de chitine », des objets cristallins aciculaires formés d'une association de chaînes copolymères de glucosamine et de N-acétyl-D-glucosamine liés par une liaison β, (1-4).

Les nanocristaux de chitine peuvent être d'origine animale ou fongique. Comme exemple de source animale, on peut citer les crustacés (crabes, crevettes, homards, etc.) et certains insectes (hannetons, scarabées, etc.). Comme exemple de source fongique de la chitine, on peut citer les champignons et les levures.

Tel que développé par la suite, la demanderesse a montré que les propriétés physico-chimiques d'une composition d'émulsion du type MIPE/HIPE stabilisée par des nanocristaux de chitine sont conditionnées par les paramètres suivants :

- les paramètres structurels des nanocristaux de chitine,
- la densité de charge à la surface des nanocristaux de chitine,
- le taux de recouvrement par les nanocristaux de chitine,
- le rapport volumique phase dispersée hydrophobe / phase continue hydrophile,
- la force ionique ou le pH de la composition d'émulsion, et
- la méthode de préparation.

Tel que développé également par la suite, la demanderesse a montré que les propriétés physico-chimiques d'une composition du type mousse stabilisée par des nanocristaux de chitine sont conditionnées par les paramètres suivants :

- le taux de recouvrement par les nanocristaux de chitine,
- la force ionique ou le pH de la composition d'émulsion.

En particulier, les nanocristaux de chitine présentent, de manière inattendue, des qualités supérieures de stabilisation (liées à la présence de fonctions acétylamine dont le caractère hydrophobe renforce le caractère amphiphile amenant ces nanocristaux plus aisément à l'interface).

Les nanocristaux peuvent encore être issus de polysaccharides choisis parmi le $\beta$-1,3-glucane, le $\beta$-1,3-xylane et le $\beta$-1,4-mannane, qui ont en commun une structure fibrillaire semblable à celle de la chitine. Ainsi, ces polysaccharides peuvent également se présenter sous forme de nanocristaux.

Ces derniers nanocristaux peuvent être d'origine végétale ou fongique. Comme exemple de source végétale, on peut notamment citer, pour le $\beta$-1,3-xylane, certaines algues. Pour le $\beta$-1,4-mannane, on peut également citer certaines algues, ainsi que l'endosperme de graines de plantes terrestres. Comme exemple de source fongique des polysaccharides, on peut notamment citer, pour le $\beta$-1,3-glucane, certains champignons et certaines levures.

Les nanocristaux peuvent encore être issus de polysaccharides choisis parmi l'amidon, $\alpha$-1,4-glucane et $\alpha$-1,6-glucane.

La composition selon l'invention peut ainsi contenir avantageusement :

(i) des nanocristaux de chitine exclusivement, ou

(ii) des nanocristaux de chitine, avantageusement chargés positivement, mélangés avec des nanocristaux issus d'au moins un autre polysaccharide (par exemple la cellulose) avantageusement chargés négativement.

Selon un mode de réalisation préféré du cas (i), la composition est stabilisée uniquement par les nanocristaux de chitine, sans ajout :

- ni d'autres composés émulsifiants ou stabilisants,
- ni d'autres particules solides, que lesdites particules solides soient fonctionnalisées ou non fonctionnalisées.

Selon le cas (ii), le ratio nanocristaux de chitine chargés positivement / nanocristaux issus de polysaccharide(s) chargés négativement, est avantageusement ajusté selon une valeur correspondant à la charge de surface souhaitée.

Ce ratio peut être choisi par exemple entre 1/99 et 99/1, de préférence entre 10/90 et 90/10, notamment parmi 1/99, 10/90, 20/80, 30/70, 40/60, 50/50, 60/40, 70/30, 80/20, 90/10 ou 99/1.

Dans ce cas, la surface des gouttes ou bulles serait avantageusement du type zwittérionique (positive et négative).

La composition peut contenir encore tout autre composé approprié à son utilisation, à sa destination finale ou à son application.

L'application de la composition peut être choisie parmi les compositions utilisables dans les domaines alimentaires, cosmétiques, pharmaceutiques, ou encore phytosanitaires.

En fonction de l'application recherchée pour la composition finale selon l'invention, la composition peut contenir par exemple, sans être aucunement limitatif, des principes actifs et des adjuvants tels que des conservateurs, des gélifiants, des solvants, des matières colorantes, etc.

*Caractéristiques dimensionnelles des nanocristaux de polysaccharide*

Les nanocristaux de polysaccharide, notamment de chitine, sont des particules solides hautement cristallines.

Ces nanocristaux de polysaccharide sont dépourvus, ou au moins pratiquement dépourvus, de partie amorphe. Ils présentent de préférence un taux de cristallinité d'au moins d'au moins 60%, et de préférence compris entre 60% et 95%.

Les nanocristaux de polysaccharide, autres que ceux de cellulose, se présentent avantageusement sous forme de bâtonnets anisotropes (Revol JF and Marchessault RH, Int. J. Biol. Macromol. (15) 1993, 325), de plaquettes ou de sphères (sphérulites) (Murray S.B. and Neville A.C. Int J. Biol. Macromol. 20 (1997) 123-130).

Par « bâtonnets anisotropes », on entend des particules orientables avec un rapport longueur / largeur supérieur à 1.

Cette morphologie peut être observée par exemple par microscopie électronique, en particulier par microscopie électronique en transmission (ou « MET »).

Ces nanocristaux ont avantageusement des largeurs variant de 2 à 50 nm, pour des longueurs maximales de l'ordre du micron.

Avantageusement, les nanocristaux de chitine ont une forme allongée anisotrope.

Les nanocristaux de chitine comportent généralement les caractéristiques dimensionnelles suivantes : (i) une longueur moyenne comprise entre 150 et 600 nm, et (ii) une largeur comprise entre 5 et 50 nm.

A titre d'exception, il est également connu des nanocristaux issus de la chitine du « rifia » (animal des grands fond) dont les caractéristiques dimensionnelles sont : (i) une longueur moyenne de l'ordre de 2200 nm, et (ii) une largeur de l'ordre de 18 nm.

Par « longueur », on entend la plus grande dimension des nanocristaux, séparant deux points situés aux extrémités de leur axe longitudinal respectif.

Par « largeur », on entend la dimension mesurée le long des nanocristaux, perpendiculairement à leur axe longitudinal respectif et correspondant à leur section maximale.

Dans des modes de réalisation préférés, les nanoparticules de chitine forment une population relativement homogène de nanocristaux dont les valeurs expérimentales de longueur suivent une distribution Gaussienne centrée sur la valeur de longueur assignée pour ladite population de nanocristaux. Dans ces modes de réalisation préférés, on peut utiliser par exemple des nanocristaux de chitine d'une « seule taille déterminée », comme cela est illustré dans les exemples.

En pratique, la morphologie et les dimensions des nanocristaux peuvent être déterminées en utilisant différentes techniques d'imagerie comme la microscopie électronique à transmission (MET) ou la microscopie à force atomique (AFM), la diffusion des rayons X ou des neutrons aux petits angles (respectivement SAXS pour « Small-Angle X-ray Scattering » et SANS pour « Small-Angle Neutron Scattering ») ou encore la diffusion dynamique de la lumière (DDL).

Avantageusement, les nanocristaux de chitine ont un rapport longueur/largeur supérieur à 1 et inférieur à 100, de préférence compris entre 5 et 30.

Un rapport longueur/largeur supérieur à 1 et inférieur à 100 englobe les rapports longueur/largeur d'au moins 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 et 99.

Par exemple, les nanocristaux de chitine peuvent être issus de :

- carapace de crabe : 240 nm sur 15 nm (Nair et Dufresne, Biomacromolecules 2003) ou 260 $\pm$ 80 nm sur 23 $\pm$ 3 nm (Belamie et al. J. Phys. Chem. 2004), ou
- carapace de crevettes : 200 à 560 nm sur 18 à 40 nm (Phonggying et al. Polymer 2007).

*Charge à la surface des nanocristaux de polysaccharide*

Pour optimiser la stabilité des compositions, les nanocristaux de polysaccharide sont avantageusement choisis en fonction de leurs caractéristiques de surface, tenant compte notamment des aspects électrostatiques.

En particulier, les nanocristaux de chitine sont chargés positivement du fait de fonctions amines en surface (en particulier un groupement N-acétylglucosamine) ; la densité de charges de surface varie ensuite en fonction du taux d'acétylation.

Concernant les caractéristiques électrostatiques de surface, les nanocristaux de chitine stabilisant l'émulsion ont avantageusement une densité de charges de surface maximale de l'ordre de 1 e.nm$^{-2}$. On note que « e » correspond à une charge élémentaire.

La densité de charge en surface peut être éventuellement choisie en fonction de la force ionique de la phase aqueuse.

L'effet déstabilisant de la densité de charge en surface peut être éventuellement annihilé par une augmentation de la force ionique dans la phase aqueuse.

Avantageusement, cette densité de charge de surface est déterminée par dosage conductimétrique.

La densité désirée de charges de surface peut être obtenue par un contrôle du taux d'acétylation des nanocristaux.

Le « degré d'acétylation » (ou « DA ») est la fraction molaire x 100 des unités N-acétyl-D-glucosamine sur le nombre total d'unités glucosamine.

Le DA peut être déterminé par différentes méthodes : titration conductimétrique, mesure par analyse élémentaire du ratio azote / carbone, spectrométries IR ou UV, RMN liquide 1 H ou solide 13C, ou par réaction enzymatique (M. Rinaudo, Progress in Polymer Science, 2006, 31, 603-632).

La désacétylation de la chitine peut s'effectuer en condition alcaline (NaOH concentré) ou par hydrolyse enzymatique en présence de chitine désacétylase.

Il est possible de désacétyler partiellement les nanocristaux de chitine par un traitement alcalin, par exemple une solution de NaOH 33% à 90°C pendant 2h à 4h avec des rendements de 85% à 90% (Y. M. Fan, T. Saito and A. Isogai, Carbohydrate Polymers, 2010, 79, 1046-1051).

Le degré d'acétylation (DA) définit la solubilité de la chaîne et donc sa nomenclature.

Pour des DA supérieurs à 50%, la chitine se présente sous une forme solide insoluble ; pour des DA inférieurs à 50%, on obtient des chaines solubles de chitosane.

Selon l'invention, ce taux d'acétylation est avantageusement compris entre 50% et 100%, de préférence d'au moins 70% (par exemple de l'ordre de 74%), de préférence encore de l'ordre de 90%.

De manière générale, les nanocristaux de polysaccharide utilisés selon l'invention sont des nanocristaux qui n'ont pas subi de modifications des fonctions en surface.

Cela englobe les nanocristaux de chitine dont les fonctions amines n'ont pas été modifiées par désacétylation.

Notamment, on utilise préférentiellement des nanocristaux de polysaccharide qui n'ont pas été fonctionnalisés ou greffés avec des groupes permettant leur réticulation subséquente, par exemple par des groupes du type méthacrylate ou diméthacrylate.

Egalement, on utilise préférentiellement des nanocristaux de polysaccharide qui n'ont pas été fonctionnalisés ou greffés par des molécules polymères, telles qu'un polyéthylène glycol, un poly(hydroxyester) ou encore un polystyrène.

De manière générale, il semblerait qu'il y ait un effet de la densité de charges de surface en absence de sel, mais que cet effet disparaisse par ajout de NaCl (qui écrante les charges et limite les répulsions électrostatiques).

*Quantité de nanocristaux de polysaccharide, autres que les nanocristaux de cellulose, et taux de recouvrement*

La composition comprend avantageusement de 0,035% à 2% en poids, de préférence encore de 0,05% à 1% en poids, de nanocristaux de polysaccharide, avantageusement de chitine, par rapport au poids total de ladite composition.

Cette proportion massique en nanocristaux de polysaccharide peut être évaluée par exemple par extrait sec de la phase aqueuse ou par dosage des sucres après hydrolyse.

On a montré selon l'invention qu'une quantité de nanocristaux de polysaccharide suffisante pour l'obtention d'un taux de recouvrement d'au moins 50%, de préférence d'au moins 60%, de préférence encore d'au moins 70%, avantageusement de l'ordre de 80%, en fonction du type de nanocristaux mis en oeuvre, est requise pour l'obtention d'une composition d'émulsion MIPE ou HIPE finale selon l'invention.

Au sens de la présente description, le « taux de recouvrement » par des nanocristaux de polysaccharide représente la proportion de la surface des gouttelettes de la phase hydrophobe dispersées dans la phase aqueuse, à l'interface huile/eau, qui est recouverte par les nanocristaux de polysaccharide.

Le taux de recouvrement « C », qui est le rapport entre (i) la surface de nanocristaux de polysaccharide présents dans la composition d'émulsion susceptible de se stabiliser à l'interface phase interne hydrophobe/phase continue hydrophile et (ii) la surface totale des gouttelettes de phase hydrophobe dans ladite composition d'émulsion, est calculé selon la formule (I) suivante :

$$C = S_p / S_d \ (I),$$

dans laquelle :

- $S_p$ représente la surface de nanocristaux de polysaccharide susceptible de se stabiliser à l'interface présents dans la composition d'émulsion, et
- $S_d$ représente la surface totale des gouttelettes de phase hydrophobe dans la composition d'émulsion.

La surface des nanocristaux est assimilée à une surface à un seul plan, en prenant l'hypothèse que les nanocristaux sont alignés sur ladite surface en un ruban plat.

En conséquence, la valeur de surface des nanocristaux peut être calculée selon la formule (II) suivante :

$$S_P = N_P L l = \frac{m_p}{h \rho_p} \ (II),$$

avec :

$$N_P = \frac{m_p}{Vp \times \rho_P} = \frac{m_p}{L \times l \times h \times \rho_P}$$

dans laquelle :

- $S_p$ représente la surface de nanocristaux de polysaccharide susceptible de se stabiliser à l'interface présents dans la composition d'émulsion,
- $N_p$ signifie le nombre de nanocristaux de polysaccharide présents dans la phase aqueuse,
- L signifie la longueur des nanocristaux de polysaccharide,
- l signifie la largeur des nanocristaux de polysaccharide,
- h signifie la hauteur des nanocristaux de polysaccharide,
- $m_p$ signifie la masse de nanocristaux de polysaccharide, et
- p signifie la densité des nanocristaux de polysaccharide.

La surface des gouttelettes est la surface à l'interface huile/eau, qui a été calculée pour chaque diamètre moyen de gouttelettes selon D(3,2).

En conséquence, la valeur de surface des gouttelettes peut être calculée selon la formule (III) suivante :

$$S_d = 4\pi R^2 \times Ng = 4\pi R^2 \times \frac{3V_{oil}}{4\pi R^3} = \frac{3V_{oil}}{R} \quad \text{(III)},$$

avec :

$$N_g = \frac{Voil}{4/3\pi R^3} \quad \text{(IV)}$$

dans laquelle :

- Ng signifie le nombre de gouttes présentes dans l'émulsion,
- $S_d$ signifie la valeur de surface des gouttelettes de phase hydrophobe,
- R signifie le rayon moyen des gouttelettes, et
- $V_{oil}$ signifie le volume total de la phase interne hydrophobe.

La valeur finale du taux de recouvrement « C » est calculée selon la formule (I) déjà mentionnée ci-dessus :

$$C = Sp / Sd \text{ (I)},$$

dans laquelle :

- $S_p$ représente la surface de nanocristaux de polysaccharide susceptible de se stabiliser à l'interface et présents dans la composition d'émulsion,
- $S_d$ représente la surface totale des gouttelettes de phase hydrophobe dans la composition d'émulsion.

Les exemples montrent encore qu'une composition sous forme de mousse peut être obtenue à partir d'une solution contenant une concentration d'au moins 6g/L de chitine.

*Force ionique et pH*

La demanderesse a également montré que la stabilité de la composition d'émulsion peut être accrue en utilisant une phase aqueuse ayant une force ionique minimale déterminée.

Comme cela est montré dans les exemples avec des nanocristaux de chitine, une stabilité optimale de l'émulsion est obtenue à partir d'un seuil minimal de valeur de force ionique de la phase aqueuse (par exemple une concentration finale à 2mM de NaCl, pour une suspension à 3g/L de nanocristaux de chitine et une concentration en HCl de 0,01 mM).

Comme cela est encore montré dans les exemples, une stabilité maximale de la composition d'émulsion est obtenue pour une valeur de force ionique correspondant à une concentration finale de NaCl de 10 à 50 mM dans ladite émulsion.

Toujours comme observé dans les exemples, l'absence de force ionique ou une augmentation de la force ionique au-delà de 10 mM tendent à une déstabilisation d'une composition du type mousse. Dans ce cas, la force ionique est avantageusement comprise entre 1 et 5 mM de NaCl.

Sans vouloir être lié par une quelconque théorie, le demandeur pense que la valeur seuil de force ionique de la phase aqueuse à partir de laquelle une stabilité optimale de l'émulsion est obtenue est celle pour laquelle les charges (contre-ions) présentes dans la phase aqueuse neutralisent les charges (ions) présentes sur les nanocristaux.

Comme cela est également montré dans les exemples, la présence de contre-ions en excès n'influe pas significativement sur les propriétés de stabilité de l'émulsion. Pour un excès massif de contre-ions, qui n'a pas été atteint dans les conditions opératoires des exemples, on peut présager une variation des conditions du fait d'une précipitation des nanocristaux sans nécessairement altérer la stabilité de l'émulsion (le phénomène d'agrégation s'est avéré plutôt favorable pour la stabilisation de l'émulsion).

A titre indicatif, selon un mode de réalisation particulier, les nanocristaux de chitine comportent avantageusement une densité de charges de surface maximale de 0,9 e.nm$^{-2}$ pour stabiliser une composition comportant une force ionique au moins égale à la force ionique équivalente à 5 mM de NaCl.

Pour une composition comportant une force ionique supérieure à la force ionique équivalente à 10 mM de NaCl, la densité de charges de surface portée par les nanocristaux de chitine semble ne plus être un paramètre pertinent pour la stabilisation effective de l'émulsion.

Une force ionique supérieure à la force ionique équivalente à 10 mM de NaCl inclut une force ionique supérieure à 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 210, 220, 230, 240, 250, 260, 270, 275, 280, 290, 300, 310, 315, 320, 325, 330, 335, 340, 345, 350, 360, 370, 375, 380, 390, 400, 410, 420, 430, 440, 450, 460, 470, 480, 490, ou supérieure à 500 mM de NaCl. De préférence, la force ionique est inférieure à une force ionique équivalent à 3 M de NaCl.

La demanderesse a aussi montré que la stabilité de la composition d'émulsion peut être accrue en utilisant une phase aqueuse ayant un pH déterminé, dont la valeur est ajustée avantageusement par la concentration en acide.

Par exemple, l'acide est choisi parmi les acides minéraux ou organiques, avantageusement parmi l'acide chlorhydrique, l'acide perchlorique, l'acide acétique, l'acide formique, l'acide chloroacétique, l'acide citrique, l'acide picrique, l'acide ascorbique et les acides gras comprenant jusqu'à 12 atomes de carbone ; l'acide chlorhydrique est préférentiellement utilisé.

Comme cela est montré dans les exemples, une stabilité maximale de la composition d'émulsion est obtenue pour une valeur de pH comprise entre 3 et 6. De même, l'augmentation du pH de 3 à 5 favorise la stabilisation de la composition sous forme de mousse.

Encore dans les exemples, il est montré que la formation de mousse est réversible en fonction du pH.

_Structure de la composition d'émulsion_

Pour définir la structure de la composition d'émulsion, on peut utiliser en particulier des caractéristiques relatives à la distribution de taille des gouttelettes lipidiques constitutives de la phase hydrophobe dispersée dans la phase hydrophile.

On peut citer par exemple sa population de gouttelettes lipidiques la plus importante en volume, dénommée encore « mode », mesurée en $\mu$m.

La distribution en taille des gouttelettes lipidiques peut être caractérisée par un diamètre dit « Sauter » ($d_{3.2}$) et une valeur $d_{4.3}$, définis respectivement par les formules suivantes :

$$d_{3.2} = \Sigma n_i d_i^3 / \Sigma n_i d_i^2$$

$$d_{4.3} = \Sigma n_i d_i^4 / \Sigma n_i d_i^3$$

où $n_i$ est le nombre de gouttelettes lipidiques de diamètre $d_i$.

Ces deux valeurs permettent d'évaluer au mieux la distribution en taille des gouttelettes lipidiques dispersées dans la phase continue hydrophile.

Cette population de gouttelettes lipidiques est par exemple déterminée par la technique de granulométrie laser.

_La phase interne_

La phase hydrophobe est choisie parmi les huiles végétales, les huiles animales, les huiles minérales, les huiles de synthèse, les solvants organiques hydrophobes et les polymères liquides hydrophobes.

La phase hydrophobe peut être choisie parmi un alcane ou un cycloalcane, substitué ou non substitué. Les exemples illustrent des modes de réalisation d'une émulsion HIPE selon l'invention respectivement avec des alcanes et des cycloalcanes.

Pour la phase hydrophobe, un alcane ayant plus de 5 atomes de carbone englobe les alcanes ayant plus de 6, 7, 8, 9, 10, 11, 12, 13, 14, 15, 16 ou plus de 17 atomes de carbone, c'est-à-dire notamment, selon la nomenclature conventionnelle, les alcanes en $C_6$-$C_{18}$ et qui sont de formule $C_n H_{2n+2}$. Lesdits alcanes peuvent être linéaires ou ramifiés.

Lesdits alcanes englobent les alcanes linéaires ou ramifiés des types hexane, heptane, octane, nonane, décane, undécane, dodécane, tridécane, tétradécane, pentadécane, hexadécane, heptadécane et octadécane.

Les alcanes substitués englobent les alcanes linéaires ou ramifiés ci-dessus dont au moins un atome d'hydrogène est substitué par un halogène choisi parmi le chlore, le brome, l'iode ou le fluor. La substitution d'au moins un atome d'hydrogène, englobe la substitution de 2, 3, 4 ou 5 atomes d'hydrogène.

Dans certains modes de réalisation, ledit cycloalcane est un cyclohexane non substitué ou substitué. Le cyclohexane peut être substitué par 1, 2, 3 ou 4 atomes d'halogène choisi parmi le chlore, le brome, l'iode ou le fluor.

La phase hydrophobe peut encore comprendre un mélange de tels alcanes, par exemple sous la forme d'une huile de paraffine.

Dans certains modes de réalisation, la phase hydrophobe comprend un ou plusieurs monomères hydrophobes polymérisables, d'un type connu.

Dans d'autres modes de réalisation, la phase hydrophobe consiste essentiellement en une composition d'un monomère hydrophobe ou d'un mélange de monomères hydrophobes. A titre illustratif, la phase hydrophobe peut consister essentiellement en une composition de monomères de styrène.

Les modes de réalisation dans lesquels la phase hydrophobe comprend, ou consiste en, un monomère hydrophobe ou une combinaison de monomères hydrophobes, sont utiles en particulier pour la fabrication de billes de matériau polymère (par polymérisation de ce(s) monomère(s)).

Dans le cas d'une mousse, la phase interne consiste en un gaz, avantageusement de l'air, de l'azote ou un gaz carbonique.

*La phase hydrophile*

Par « phase hydrophile » ou « phase aqueuse », on entend un liquide immiscible avec la phase hydrophobe. On utilise de préférence une phase hydrophile miscible avec l'eau. La phase hydrophile peut être de l'eau, comme cela est illustré dans les exemples.

La phase hydrophile peut être un solvant hydrophile, de préférence un solvant portant des groupes hydroxyle, tels que des glycols. Pour la phase hydrophile, les glycols englobent le glycérol et les polyéthylène glycols.

La phase hydrophile peut également contenir des agents hydrosolubles texturants, notamment épaississants ou viscosifiants, tels que des polysaccharides (par exemple dextran ou xanthane, ce dernier étant très utilisé dans les applications alimentaires).

La phase hydrophile peut être constituée, en partie ou totalement, par un liquide organique choisi parmi un alcool tel que l'éthanol, ou encore l'acétone.

La phase hydrophile peut comprendre un seul liquide ou un mélange de plusieurs liquides.

L'homme du métier peut aisément adapter la constitution de la phase hydrophile, notamment en fonction de l'émulsion MIPE ou HIPE finale qui est désirée.

Dans certains modes de réalisation, la phase hydrophile peut comprendre diverses substances additionnelles ou combinaison de substances additionnelles utiles pour l'application industrielle qui est recherchée pour l'émulsion MIPE ou HIPE, tels que des principes actifs de médicament.

Dans certains modes de réalisation, la phase hydrophile comprend un ou plusieurs monomères hydrophiles, qui peuvent être subséquemment polymérisés au sein de l'émulsion MIPE ou HIPE.

Dans certains modes de réalisation, la phase hydrophile comprend un ou plusieurs monomères hydrophiles polymérisables, d'un type connu.

Dans d'autres modes de réalisation, la phase hydrophile consiste essentiellement en une composition d'un monomère hydrophile ou d'un mélange de monomères hydrophiles. A titre illustratif, la phase hydrophile peut consister essentiellement en une composition de monomères hydrophiles de type acrylate.

Les modes de réalisation dans lesquels la phase hydrophile comprend, ou consiste en, un monomère hydrophile ou une combinaison de monomères hydrophiles, sont utiles en particulier pour la fabrication de matériau polymère poreux.

*Procédé **d'obtention***

La présente invention concerne aussi le procédé d'obtention de la composition présentée ci-dessus.

Pour la fabrication d'une mousse par dispersion d'une phase gazeuse dans la phase hydrophile, il est possible de se référer par exemple au protocole décrit dans le document Lucassen, J. (1981) Lucassen-Reijnders, E. H. ed. Anionic Surfactants - Physical Chemistry of Surfactant Action NY, USA: Marcel Dekker, dans le document Alargova et al. - « Foam superstabilization by polymer microrods », Langmuir, vol. 20(24), 2004, 10371-10374, ou dans le document Blanco E. et al., « Stability and Viscoelasticity of Magneto-Pickering Foams », Langmuir 29, (2013), 10019-10027.

De manière générale, plusieurs méthodes sont possibles pour disperser un gaz dans un liquide, à savoir en substance, insuffler le gaz dans le liquide, le fabriquer *in situ,* battre le liquide en présence du gaz ou encore mélanger un gaz et un liquide en leur faisant traverser un matériau poreux.

Dans ce cas, on obtient avantageusement une mousse hydrophile liquide particulaire, de préférence une mousse dite « sèche » telle que décrite ci-dessus.

Selon cette application, les nanocristaux de chitine ont l'intérêt spécifique de conférer des propriétés de moussabilité particulièrement intéressantes à une solution.

La « moussabilité » ou le « pouvoir moussant » d'une solution est une mesure qualitative de sa capacité à produire une mousse lorsqu'elle est secouée ou lorsque des bulles y sont injectées.

De manière surprenante, la demanderesse a encore montré que des émulsions MIPE ou HIPE du type huile-dans-l'eau possédant une haute teneur en phase dispersée hydrophobe, supérieure à 50% voire supérieure à 75% du volume total de l'émulsion, peuvent être obtenues de manière « séquentielle » (désigné encore en « deux étapes »), à partir d'émulsions du type Pickering stabilisées par des nanocristaux de polysaccharide autre que la cellulose.

Les émulsions du type Pickering sont connues dans l'état de la technique, et consistent en des émulsions qui sont stabilisées par des particules en suspension colloïdale localisées à l'interface huile/eau.

En général, les émulsions de Pickering sont dépourvues d'agent tensio-actif conventionnel. Dans certains modes de réalisation, une émulsion de Pickering peut contenir un ou plusieurs agents tensio-actifs conventionnels, mais en quantité insuffisante pour stabiliser une émulsion.

Les émulsions de Pickering, stabilisées par des nanocristaux de polysaccharide autre que la cellulose, qui sont utilisées comme produit de départ pour l'obtention des émulsions MIPE ou HIPE du genre huile-dans-l'eau divulguées dans la présente description, sont spécifiques à la présente invention, et leur procédé de préparation est spécifié en détail plus loin.

Plus précisément, la demanderesse a montré que, de manière surprenante, des émulsions MIPE ou HIPE du type précité peuvent être obtenues lorsque l'on utilise comme composition de départ une émulsion huile-dans-l'eau de Pickering stabilisée par des nanocristaux de chitine.
Notamment, on a montré selon l'invention que des émulsions HIPE sont obtenues car les compositions d'émulsion de Pickering stabilisées par des nanocristaux de chitine permettent ensuite de dépasser l'état d'empaquetage compact des gouttes de phase interne hydrophobe (aussi appelé état de « close packing »), c'est-à-dire d'obtenir un pourcentage de phase interne supérieur à 75%.

D'autre part, la demanderesse montre encore que les nanocristaux de chitine ont la propriété spécifique et inattendue de permettre directement l'obtention d'une composition d'émulsion à phase interne élevée (procédé dit « direct »), sans nécessiter de passer par l'intermédiaire d'une émulsion de type Pickering.

Tel qu'illustré par la suite, dans le cas des compositions d'émulsion, la voie « directe » intègre la totalité de la phase hydrophile introduite, alors que la voie « séquentielle » a tendance à exclure de la phase hydrophile ce qui augmente le pourcentage de phase interne pour les MIPEs. Au-delà de 74% de phase interne, les pourcentages de phase interne sont identiques dans les deux procédés.

Le procédé selon l'invention comprend avantageusement les opérations suivantes :

a) une étape d'incorporation de nanocristaux d'un polysaccharide autre que la cellulose, avantageusement de chitine, dans une phase hydrophile,
b) une opération de fourniture (i) de ladite phase hydrophile contenant les nanocristaux de polysaccharide et (ii) d'une phase destinée à constituer la phase interne (une phase liquide hydrophobe ou une phase gazeuse, selon le cas),
c) une opération de formation de ladite composition par dispersion de ladite phase interne dans ladite phase hydrophile.

De manière générale, on a montré dans les exemples que le procédé d'obtention d'émulsions HIPE selon l'invention permet la préparation de compositions d'émulsion avec une haute teneur en phase interne hydrophobe, ayant jusqu'à plus de 95% en volume de phase interne hydrophobe.

On a aussi montré que des émulsions MIPE ou HIPE préparées selon le procédé de l'invention et ayant un rapport volumique phase interne hydrophobe / phase dispersée hydrophile supérieur à 60% peuvent se présenter sous la forme d'un gel solide.

On a montré dans les exemples que des compositions d'émulsion MIPE ou HIPE préparées par le procédé selon l'invention sont stables pendant une longue période de temps, en l'occurrence plusieurs mois, y compris lorsqu'elles sont conservées à une température d'environ 20°C.

De plus, on a montré que des émulsions MIPE ou HIPE obtenues conformément au procédé de l'invention ont une excellente capacité de résistance à la compression.

Egalement, la demanderesse a montré la réversibilité de la rupture d'une émulsion HIPE selon l'invention, par exemple par cisaillement (par exemple par agitation forte) ou compression (par exemple par centrifugation forte). Une émulsion HIPE selon l'invention a donc la propriété de se reformer à nouveau après une rupture.

Par une étude des émulsions HIPE de l'invention par microscopie confocale à balayage, on a observé que les gouttes d'huile dispersées dans la phase continue aqueuse se déforment avec les rapports croissants phase interne hydrophobe / phase dispersée hydrophile, jusqu'à se conformer en polyèdres, ce qui minimise le volume occupé par la phase continue aqueuse.

On a aussi montré qu'une émulsion HIPE selon l'invention peut être soumise à un traitement pour réaliser une émulsion sèche, par exemple lorsque la phase interne hydrophobe est constituée d'une huile moins volatile que l'eau, polymérisable ou non lyophilisable et qu'en conséquence seule la phase continue aqueuse est éliminée par séchage ou lyophilisation.

On a aussi montré qu'une émulsion HIPE selon l'invention peut être utilisée pour réaliser des mousses sèches, par exemple (i) soit par lyophilisation de ladite émulsion lorsque les deux phases sont lyophilisables, (ii) soit, lorsque la phase dispersée hydrophobe comprend des monomères polymérisables, par polymérisation desdits monomères puis élimination de la phase continue aqueuse.

*Fourniture des nanocristaux de polysaccharide*

Certains polysaccharides, notamment la chitine, se composent d'une partie dite « amorphe », alors qu'une seconde partie est « cristalline ».

Les nanocristaux de polysaccharide sont avantageusement issus de la partie cristalline isolée, par élimination de la partie amorphe du polysaccharide.

A partir de la matière première choisie, les nanocristaux de polysaccharide sont préparés par un procédé avantageusement choisi parmi l'un des procédés suivants : fractionnement mécanique, hydrolyse chimique ménagée, et dissolution / recristallisation.

Par « fractionnement mécanique », on entend une opération classique d'homogénéisation haute pression.

Par « hydrolyse chimique ménagée », on entend un traitement par un composé chimique acide du polysaccharide, dans des conditions assurant l'élimination de sa partie amorphe.

Le composé chimique acide est avantageusement choisi parmi l'acide sulfurique ou l'acide chlorhydrique.

Tel que décrit dans les exemples ci-après, selon le type d'acide, la température et le temps d'hydrolyse, la charge de surface peut être modulée.

Par « dissolution/recristallisation », on entend un traitement acide ou basique ou par un solvant, par exemple acide phosphorique, urée/NaOH, liquides ioniques, etc., suivi d'une recristallisation. Un tel procédé est par exemple décrit dans A. Osorio-Madrazoa et al., Carbohydrate Polymers 83(4), (2011), 1730-1739.

Avant leur intégration dans la composition, les nanocristaux de polysaccharide obtenus peuvent être soumis à un procédé de post-modification, à l'issue duquel leur densité de charges de surface et/ou leur hydrophilicité sont modifiées, à condition que la post-modification ne génère pas de nanocristaux uniquement hydrophobes.

Cette post-modification vise à optimiser les caractéristiques de surface des nanocristaux, notamment en fonction de l'émulsion dans laquelle ils sont introduits, de manière à optimiser sa stabilisation.

Pour modifier la densité de charges de surface de la chitine, le procédé de post-modification consiste avantageusement en un traitement au NaOH pendant 2 à 4h décrit précédemment (Y. M. Fan, T. Saito and A. Isogai, Carbohydrate Polymers, 2010, 79, 1046-1051).

Pour modifier l'hydrophobicité et la charge de surface de nanocristaux de polysaccharide neutre, le procédé de post-modification vise avantageusement à générer des groupements N-acétylglucosamine dans ces nanocristaux de polysaccharide.

De manière alternative et préférée, les nanocristaux de polysaccharide obtenus ne présentent aucune modification de charge de surface, tel que développé précédemment.

A titre indicatif, des nanocristaux de chitine fournis à l'étape a) du procédé d'obtention d'une émulsion MIPE ou HIPE selon l'invention sont avantageusement obtenus par un procédé de fabrication à partir de chitine.

L'étape d'incorporation des nanocristaux de polysaccharide dans la phase aqueuse correspond aux étapes mises en oeuvre pour l'incorporation de particules colloïdales lors de la fabrication d'émulsions de Pickering.

*Procédé « séquentiel » ou en « deux étapes »*

Selon un mode de réalisation dit « séquentiel » ou en « deux étapes », l'opération c) pour la formation de la composition d'émulsion par dispersion de la phase hydrophobe dans la phase hydrophile se compose de deux étapes successives :

c.1) une étape de dispersion de ladite phase hydrophile dans ladite phase hydrophobe, avec un rapport volumique phase hydrophobe / phase hydrophile d'au moins 5/95, pour l'obtention d'une émulsion huile-dans-eau intermédiaire du type Pickering, suivi de
c.2) une étape d'obtention de la composition d'émulsion d'intérêt possédant un pourcentage de phase interne supérieur à 50%, le cas échéant du type MIPE ou HIPE, comprenant :

c.2.1) une étape d'ajout d'un volume de phase hydrophobe à la composition d'émulsion intermédiaire obtenue à l'étape c.1), et agitation du mélange ainsi obtenu, et/ou
c.2.2) une étape de concentration de la composition d'émulsion obtenue à l'étape c.1), par retrait d'au moins une partie de ladite phase hydrophile.

*Fourniture d'une émulsion huile-dans-l'eau intermédiaire du type Pickering, stabilisée par des nanocristaux de polysaccharide*

L'émulsion intermédiaire de Pickering utilisée pour l'obtention d'une émulsion MIPE ou HIPE selon l'invention consiste en une composition sous forme d'une émulsion comprenant une phase hydrophobe dispersée dans une phase aqueuse, et contenant des particules émulsifiantes (ou autrement dit « particules émulsionnantes ») comprenant des nanocristaux d'un polysaccharide autre que la cellulose.

Comme déjà précisé, l'émulsion de Pickering est du genre « huile dans eau ».

Les nanocristaux mis en oeuvre, ainsi que les phases hydrophobe et hydrophile utilisées, sont tels que développés ci-dessus.

L'émulsion de Pickering est ainsi stabilisée au moins par des nanocristaux d'un polysaccharide autre que la cellulose, avantageusement des nanocristaux de chitine.

Selon un mode de réalisation préféré, la composition d'émulsion de Pickering intermédiaire est stabilisée uniquement par les nanocristaux d'un polysaccharide autre que la cellulose, avantageusement des nanocristaux de chitine, sans ajout d'autre composé émulsifiant ou stabilisant.

La composition comprend avantageusement de 0,5 % en poids de nanocristaux d'un polysaccharide autre que la cellulose, avantageusement des nanocristaux de chitine, par rapport au poids total de ladite émulsion de Pickering.

Comme développé ci-dessus, on a montré selon l'invention qu'une quantité de nanocristaux d'un polysaccharide autre que la cellulose, suffisante pour l'obtention d'un taux de recouvrement d'au moins 50%, en fonction du type de nanocristaux mis en oeuvre, est requise pour la préparation d'une composition d'émulsion de Pickering qui est adaptée à l'obtention d'une composition d'émulsion MIPE ou HIPE finale selon l'invention.

A l'étape c.1), l'émulsion huile-dans-l'eau du type Pickering présente avantageusement un rapport volumique phase hydrophobe / phase hydrophile avantageusement d'au moins 5/95, et de préférence d'au plus 50/50, voire d'au plus 60/40.

Par « au moins 5/95 », on entend une valeur minimale de 5 pour la phase hydrophobe dans le rapport volumique.

Par « au plus 50/50 » ou « au plus 60/40 », on entend la valeur maximale de 50 ou 60, respectivement, pour la phase hydrophobe dans le rapport volumique.

Dans ce cadre, le rapport volumique phase hydrophobe / phase hydrophile est avantageusement choisi parmi 5/95, 10/90, 15/85, 20/80, 25/75, 30/70, 35/65, 40/60, 45/55, 50/50, 55/45 ou 60/40.

Cette émulsion huile-dans-l'eau du type Pickering selon l'invention possède avantageusement un pourcentage de phase interne inférieur ou égal à 50%.

Dans ce cadre, l'émulsion huile-dans-l'eau du type Pickering comporte avantageusement un pourcentage de phase interne compris entre 5 et 30%.

*Procédé d'obtention de la composition d'émulsion de Pickering*

Le procédé pour la fabrication de la composition d'émulsion intermédiaire de Pickering, comprend avantageusement les étapes suivantes :

(a) la fourniture de nanocristaux d'un polysaccharide autre que la cellulose, avantageusement des nanocristaux de chitine, tels que définis ci-dessus, puis
(b) l'incorporation desdits nanocristaux dans la phase aqueuse de ladite composition, dans une quantité massique apte à générer un taux de recouvrement d'au moins 50%, de préférence d'au moins 80%, dans ladite émulsion intermédiaire de Pickering et à stabiliser ladite émulsion intermédiaire.

Les étapes générales pour la fabrication de l'émulsion intermédiaire peuvent être réalisées selon des procédures classiques, notamment employées pour la fabrication d'une émulsion de Pickering.

Il pourra en particulier se référer au document Tzoumaki et al., « Oil-in water emulsions stabilized by chitin nanocrystal particles », Food Hydrocolloids 25 (2011) 1521-1529.

En particulier, l'étape d'incorporation des nanocristaux de polysaccharide dans la phase aqueuse correspond aux étapes mises en oeuvre pour l'incorporation de particules colloïdales lors de la fabrication d'émulsions de Pickering.

La dispersion de la phase hydrophobe dans la phase hydrophile (contenant les nanocristaux stabilisateurs) peut être réalisée par toute technique de réalisation d'une émulsion connue par l'homme du métier.

On peut ainsi par exemple employer une technique d'obtention d'émulsion par ultrasons, comme cela est réalisé de manière conventionnelle. On peut aussi employer une technique d'obtention d'émulsion par agitation à l'aide d'un dispositif disperseur homogénéiseur du type rotor-stator, par exemple un dispositif rotor-stator connu sous le nom de Ultraturrax™, bien connu par l'homme du métier.

A titre illustratif, on peut obtenir une émulsion de Pickering stabilisée par des nanocristaux de chitine en soumettant un mélange (i) phase hydrophile / (ii) phase hydrophobe (ledit mélange comprenant la quantité appropriée de nanocristaux de chitine) à une étape d'homogénéisation par ultrasons pendant une durée de quelques secondes à quelques minutes selon la puissance du dispositif et le volume d'émulsion.

Egalement à titre illustratif, on peut obtenir une émulsion de Pickering stabilisée par des nanocristaux de chitine en soumettant un mélange (i) phase hydrophile / (ii) phase hydrophobe (ledit mélange comprenant la quantité appropriée de nanocristaux de chitine) à une étape d'homogénéisation à l'aide d'un dispositif rotor / stator du type Heidolph (Roth - Marque déposée) à une vitesse d'au moins 5000 tr/min (rpm) pendant une durée de 1 à 3 minutes.

*Obtention de la composition d'émulsion du type à moyenne phase interne (MIPE) ou à haute phase interne (HIPE) partant de l'émulsion intermédiaire de Pickering*

Si la condition de stabilité de l'émulsion intermédiaire de Pickering est remplie (étape c.1)), le procédé peut être poursuivi par la ou les étapes pour former l'émulsion MIPE ou HIPE (étape c.2)).

Selon l'invention, l'obtention de l'émulsion MIPE/HIPE peut être obtenue :

c.2.1) par ajout d'un volume de phase hydrophobe à la composition d'émulsion intermédiaire obtenue à l'étape c.1), et/ou

c.2.2) par concentration de la composition d'émulsion intermédiaire obtenue à l'étape c.1), par retrait d'au moins une partie de ladite phase hydrophile.

L'obtention de l'émulsion HIPE nécessite d'atteindre une concentration en gouttes hydrophobes dépassant le seuil de « close packing », ou encombrement maximum théorique de sphères de taille identique, correspondant à un pourcentage de phase interne supérieur à 74-75%.

A cet égard, sans être limitatif, deux voies sont envisageables :

- une taille variable de gouttelettes de la phase interne, et
- un gonflement des gouttelettes de la phase interne, puis leurs déformations.

Les Exemples ci-après montrent que l'émulsion de Pickering selon l'invention permet l'obtention d'émulsion HIPE d'intérêt, dont le pourcentage de phase interne est supérieur à 74-75%.

*Ajout d'un volume de phase hydrophobe*

Selon un premier mode de réalisation, le procédé peut être poursuivi par l'ajout de la phase hydrophobe pour former l'émulsion MIPE ou HIPE (étape c.2.1)).

Pour l'obtention de l'émulsion MIPE ou HIPE selon l'invention, à partir d'une émulsion intermédiaire de Pickering préparée comme décrit ci-dessus, on ajoute avantageusement une quantité désirée de phase hydrophobe à ladite émulsion avant de réaliser une agitation du mélange émulsion de Pickering / phase hydrophobe ajoutée.

Cette phase hydrophobe ajoutée peut être identique, ou différente, par rapport à la phase hydrophobe incorporée pour former l'émulsion de Pickering.

De manière surprenante, le demandeur a montré que la réalisation d'une simple agitation avec un dispositif homogénéiseur (p. ex. Ultraturrax™) du mélange émulsion intermédiaire de Pickering / phase hydrophobe ajoutée permet l'obtention directe d'une émulsion MIPE ou HIPE.

Comme cela est montré dans les exemples, dans une émulsion MIPE ou HIPE selon l'invention, la valeur du rapport volume de phase dispersée hydrophobe / volume d'émulsion (et donc également la valeur du rapport volume de phase dispersée hydrophobe / volume de phase continue hydrophile) dépend directement du volume de phase hydrophobe ajoutée à l'émulsion de Pickering de départ.

Comme cela est également montré dans les exemples, il ne semble pas exister de limite spécifique à la valeur du rapport volumique phase dispersée hydrophobe / phase continue aqueuse dans l'émulsion HIPE ainsi obtenue.

Dans une émulsion MIPE ou HIPE selon l'invention, la valeur du rapport volumique de phase dispersée hydrophobe / émulsion est déterminable à l'avance, en fonction du volume de phase hydrophobe ajouté à l'émulsion de Pickering de départ pour les conditions optimum d'agitation.

L'étape d'agitation du mélange émulsion de Pickering / phase hydrophobe ajoutée peut être réalisée aisément à l'aide d'un dispositif homogénéiseur / disperseur conventionnel, par exemple un dispositif d'agitation du type Ultraturrax™.

A titre illustratif, lorsqu'on utilise un dispositif d'agitation du type Ultraturrax™, l'émulsion HIPE peut être obtenue en agitant pendant une durée d'au moins 15 secondes à une vitesse de rotation d'au moins 5000 tours par minute, de préférence au moins 5500 tours par minute pour un contenant de 4 mL.

L'homme du métier adapte les conditions de l'étape d'agitation du mélange sur la base des indications de la présente description et de ses connaissances générales dans le domaine de la fabrication de compositions d'émulsion.

Pour l'étape d'agitation du mélange, une durée d'au moins 15 secondes englobe les durées d'au moins 20, 25, 30, 35, 40, 45, 50, 55, 60, 65, 70, 75, 80, 90, 95, 100, 1105, 110, 115, 120, 125, 130, 135, 140, 145, 150 secondes.

Le cas échéant, l'étape d'agitation peut avoir une durée supérieure à 200 secondes, bien que cela ne soit pas utile pour l'obtention de l'émulsion HIPE finale.

Pour l'étape d'agitation, une force d'agitation d'au moins 1000 tours par minute englobe les forces d'agitation d'au moins 1050, 1100, 1150, 1200, 1250, 1300, 1350, 1400, 1450, 1500, 1550, 1600, 1650, 1700, 1750, 1800, 1850, 1900, 2000, 2100, 2200, 2300, 2400, 2500, 2600, 2700, 2800, 2900, 3000, 3100, 3200, 3300, 3400, 3500, 4000, 4100, 4200, 4300, 4400, 4500, 4600, 4700, 4800, 4900, 5000, 5100, 5200, 5300, 5400, 5500, 5600, 5700, 5800, 5900, 6000, 6100, 6200, 6300, 6400, 6500, 6600, 6700, 6800, 6900, 7000, 7100, 7200, 7300, 7400, 7500,7600, 7700, 7800, 7900, 80000, 8100, 8200, 8300, 8400, 8500, 8600, 8700, 8800, 8900, 9000, ou d'au moins 10000 tours par minutes.

Le cas échéant, une force d'agitation supérieure à 15000 tours par minute peut être appliquée, bien que cela ne soit pas utile pour l'obtention de l'émulsion HIPE finale.

De préférence, dans les conditions générales d'agitation définies ci-dessus, la force d'agitation est inférieure à 20000 tours par minute, afin d'éviter d'altérer la structure de l'émulsion. La force d'agitation peut être aisément adaptée par l'homme du métier à la lumière du contenu de la présente description, et le cas échéant de ses connaissances générales. En particulier, la force d'agitation peut être adaptée par l'homme du métier en fonction de la viscosité de l'émulsion de Pickering de départ, et en fonction de l'accroissement de viscosité pendant la préparation de l'émulsion HIPE, qui dépend notamment de la viscosité de la phase hydrophobe qui est ajoutée.

Dans certains modes de réalisation, l'étape d'agitation avec un dispositif du type Ultraturrax™ peut être réalisée selon deux phases, respectivement une première phase au cours de laquelle on applique une première force d'agitation et une seconde phase au cours de laquelle on applique une seconde force d'agitation.

A titre illustratif, on peut réaliser l'étape d'agitation avec (i) une première phase d'agitation à 11000 tours par minute et (ii) une seconde phase d'agitation à 15000 tours par minute, par exemple avec une durée approximativement identique pour la première et la seconde phase d'agitation.

Avantageusement, l'étape d'agitation du mélange émulsion de Pickering / phase hydrophobe ajoutée est réalisée à température ambiante, c'est-à-dire en général à une température allant de 15°C à 25°C, et le plus souvent allant de 18°C à 23°C.

*Concentration de la composition d'émulsion*

Selon un second mode de réalisation, le procédé peut être poursuivi par une étape de concentration, pour former l'émulsion MIPE ou HIPE (étape c2.2)).

Cette étape de concentration conduit à retirer la phase continue hydrophile par une technique adaptée, choisie par exemple parmi :

- le crémage/sédimentation par gravité,
- la centrifugation (par exemple 2000g pendant 10 minutes),
- la filtration (avantageusement système à membrane poreuse classique ou d'ultrafiltration en continu),
- les méthodes osmotiques,
- les méthodes de cryo-concentration ou le séchage (dans des conditions pour lesquelles seule la phase continue est évaporée).

Comme cela est montré dans les exemples, dans une émulsion MIPE ou HIPE selon l'invention, la valeur du rapport volume de phase dispersée hydrophobe / volume d'émulsion dépend en particulier :

- de la concentration en chacun des constituants (phase hydrophile, phase hydrophobe, nanocristaux),
- du procédé d'obtention de l'émulsion (ultrason, rotor stator, etc.), et
- des conditions utilisées (vitesse, temps, température, énergie, volume, etc.).

Les paramètres de ces techniques seront adaptés en fonction de l'échantillon.

De tels procédés sont par exemple décrits dans les documents suivants : « Emulsions: Theory and Practice », Paul Becher Third Edition, Oxford University Press 2001 (ISBN 0-8412-3496-5) ou « High internal phase emulsions (HIPEs) - Structure, properties and use in polymer preparation », Cameron NR; Sherrington DC, BIOPOLYMERS LIQUID CRYSTALLINE POLYMERS PHASE EMULSION, ADVANCES IN POLYMER SCIENCE, Volume: 126, Pages: 163-214, 1996.

*Procédé direct*

De manière inattendue, les inventeurs ont observé que les nanocristaux de chitine permettent l'obtention d'une émulsion à phase interne élevée, en mélangeant directement la phase hydrophobe avec la phase hydrophile (contenant les nanocristaux) dans des proportions appropriées.

La dispersion de la phase hydrophobe dans la phase hydrophile peut être réalisée par toute technique de réalisation d'une émulsion connue par l'homme du métier.

On peut ainsi par exemple employer une technique d'obtention d'émulsion par ultrasons, comme cela est réalisé de manière conventionnelle. On peut aussi employer une technique d'obtention d'émulsion par agitation à l'aide d'un dispositif disperseur homogénéiseur du type rotor-stator, par exemple un dispositif rotor-stator connu sous le nom de Ultraturrax™, bien connu par l'homme du métier.

A titre illustratif, on peut obtenir une émulsion selon l'invention, stabilisée par des nanocristaux de chitine, en soumettant un mélange (i) phase hydrophile / (ii) phase hydrophobe (ledit mélange comprenant la quantité appropriée de nanocristaux de chitine) à une étape d'homogénéisation à l'aide d'un dispositif rotor / stator du type Heidolph (Roth - Marque déposée) à une vitesse de 8000 tr/min (rpm), le temps d'avoir une émulsion homogène.

*Applications industrielles d'une compositions selon l'invention*

Comme cela a déjà été mentionné précédemment dans la présente description et est illustré dans les exemples, une émulsion MIPE ou HIPE selon l'invention peut être obtenue dans l'objectif de préparer une mousse solide ou encore une émulsion solide, par exemple par simple lyophilisation de l'émulsion MIPE ou HIPE.

De manière générale, le produit issu de la composition selon l'invention est avantageusement choisi parmi une émulsion sèche, une mousse solide, un matériau polymère poreux ou des billes de matériau polymère

Pour la préparation d'une mousse solide, on utilise préférentiellement une phase hydrophobe qui peut être évaporée par lyophilisation. Ainsi, en soumettant une émulsion MIPE ou HIPE de l'invention à une étape de lyophilisation, on évapore à la fois la phase hydrophile et la phase hydrophobe, de manière à obtenir une mousse formée d'un réseau polysaccharidique, ledit réseau polysaccharidique résultant des nanocristaux de polysaccharide localisés à l'interface phase hydrophobe/phase hydrophile.

Notamment, les exemples illustrent la fabrication d'un matériau de mousse de chitine, par simple lyophilisation d'une émulsion HIPE selon l'invention.

En particulier et de manière non-limitative, les émulsions de type MIPE donnent des structures alvéolaires sphériques, et les émulsions de type HIPE donnent des structures alvéolaires non-sphériques.

La mousse solide peut être utilisée en tant que support solide dans diverses applications industrielles, y compris comme matériau d'isolation thermique ou phonique, ou encore comme biomatériau support.

Le produit résultant, c'est à dire la mousse polysaccharidique, possède une grande surface spécifique de matériau polysaccharidique, et peut être utilisé notamment comme support de principe(s) actif(s), par exemple comme support de principe(s) actif(s) pharmaceutiques, humains ou vétérinaires.

A titre illustratif, de tels supports d'intérêt pharmaceutique peuvent être obtenus lorsque le ou les principe(s) actif(s) sont ajouté(s) au préalable à l'émulsion MIPE ou HIPE, soit dans la phase hydrophobe, soit dans la phase hydrophile, en fonction des caractéristiques d'hydrophilicité du ou des principe(s) actif(s) considéré(s).

Dans certains modes de réalisation, lesdits supports polysaccharidiques peuvent comprendre à la fois (i) un ou plusieurs principe(s) actif(s) hydrophobe(s), (ii) un ou plusieurs principe(s) actif(s) hydrophile(s) et le cas échéant (iii) un ou plusieurs principe(s) actif(s) amphiphile(s).

Dans ces modes de réalisation, chaque principe actif peut être ajouté (i) soit dans l'une des phases hydrophile ou hydrophobe utilisée pour la préparation de la composition d'émulsion, (ii) soit dans l'émulsion de Pickering utilisée pour obtenir l'émulsion MIPE ou HIPE finale dans le cas d'un procédé « séquentiel », (iii) soit encore dans la phase hydrophobe qui est ajoutée à l'étape c) du procédé « séquentiel » pour obtenir l'émulsion MIPE ou HIPE finale.

L'invention a donc encore pour objet un procédé de préparation d'une mousse solide de polysaccharide comprenant les étapes suivantes :

a) fournir une émulsion MIPE ou HIPE telle que définie dans la présente description, de préférence une émulsion MIPE ou HIPE obtenue selon le procédé spécifié dans la présente description,
b) éliminer la phase hydrophile et la phase hydrophobe de ladite émulsion MIPE ou HIPE par évaporation, de préférence par lyophilisation, afin d'obtenir la mousse solide particulaire.

Une émulsion MIPE ou HIPE selon l'invention peut être également utilisée pour la fabrication d'une émulsion sèche, par évaporation de la phase hydrophile, par exemple par lyophilisation, et maintien de la phase hydrophobe. Dans ces modes de réalisation, la phase hydrophobe peut contenir une ou plusieurs substance(s) d'intérêt, par exemple un ou plusieurs principe(s) actif(s) d'intérêt pharmaceutique.

Une émulsion MIPE ou HIPE selon l'invention peut également être utilisée pour la fabrication de matériaux polymères poreux, principalement par addition de monomères hydrophiles polymérisables dans la phase aqueuse, puis polymérisation *in situ desdits* monomères hydrophiles.

Dans d'autres aspects, une émulsion MIPE ou HIPE selon l'invention peut être utilisée pour la fabrication de billes de matériau polymère, principalement par addition de monomères hydrophobes dans la phase dispersée hydrophobe, puis polymérisation desdits monomères.

Les matériaux polymères peuvent être utilisés comme matériau pour la fabrication de dispositifs médicaux y compris de matériau support pour des principes actifs physiologiquement actifs, ou encore de matériau support pour des prothèses médicales.

Les techniques d'obtention de matériaux polymères, soit des blocs de matériau polymère poreux, soit des billes de matériau polymère, à partir de différents types d'émulsion, sont connus en elles-mêmes dans l'état de la technique.

Dans certains modes de réalisation, lesdits monomères d'intérêt sont déjà présents dans la phase continue hydrophile ou dans la phase dispersée hydrophobe qui est utilisée pour l'obtention de la composition d'émulsion selon l'invention.

Dans d'autres modes de réalisation du procédé « séquentiel », lesdits monomères d'intérêt sont présents dans la phase hydrophobe qui est ajoutée à l'émulsion de Pickering de départ, à l'étape de réalisation de l'émulsion MIPE ou HIPE proprement dite.

Dans encore d'autres modes de réalisation, lesdits monomères d'intérêt sont ajoutés ultérieurement, à l'émulsion MIPE ou HIPE qui a déjà été obtenue.

Dans encore d'autres modes de réalisation, les monomères d'intérêt peuvent être ajoutés successivement à différentes étapes, dans le procédé d'obtention de l'émulsion MIPE ou HIPE selon l'invention et/ou après obtention de la composition d'émulsion MIPE ou HIPE selon l'invention.

Dans certains modes de réalisation, le ou les polymères sont utilisés en combinaison avec un ou plusieurs agents de réticulation.

Pour polymériser le ou les polymères d'intérêt, on ajoute classiquement un ou plusieurs composés initiateurs de la polymérisation appropriés.

A titre illustratif, l'utilisation d'émulsions, y compris d'émulsions HIPE huile-dans-l'eau, pour la fabrication de matériaux polymères, est par exemple décrit dans la demande PCT n° WO 2009/013500 ou encore dans les brevets US 6,218,440 et US 4,472,086.

La présente invention est en outre illustrée, sans y être limitée, par les exemples suivants.

**EXEMPLE** : **Préparation d'une émulsion huile-dans-l'eau stabilisée par des nanocristaux** de chitine

A. Protocoles

Protocole 1 : Préparation de nanocristaux de chitine

Le procédé d'obtention des nanocristaux de chitine est décrit dans les documents « In vitro chiral nematic ordering of chitin crystallites », Revol, J.-F. ; Marchessault, R.H. ; Int. Journal of Biological Macromolecules, 1993, 15, 329-335, et « Structure and chirality of the nematic phase in chitin suspensions » Belamie, E ; Davidson, P ; Giraud-Guille, M.M. Journal of Physical Chemistry B, 2004, 108 (39), 14991-15000.

Plus précisément, 4 g de chitine dans 80 mL d'HCl 3N, sont maintenus à ébullition et sous agitation durant 90 minutes.

La suspension est ensuite diluée, lavée à l'eau ultrapure par centrifugations successives à 10000 tr/min pendant 20 minutes, et dialysée contre du HCl 0,01 mM pendant cinq jours.

La dispersion finale, constituée de nanocristaux de chitine, est soumise à un traitement ultrason, filtrée sur

5μm puis 1,2μm puis conservée à 4°C.

Protocole 2 : Microscopie électronique en transmission (TEM)

20 μL d'une suspension aqueuse de nanocristaux de chitine (0,0025% poids/volume) sont déposés sur une grille carbonée pour microscopie électronique.

Après 2 min, l'excès de solvant est absorbé et l'échantillon est marqué par ajout de 20μL d'acétate d'uranyle (2% dans l'eau) ; l'excès est absorbé après 2 min.

Cette grille pour microscopie électronique est ensuite séchée dans une étuve à 40°C.

Les grilles ont ensuite été observées avec un microscope électronique en transmission de marque JEOL (80kV).

Protocole 3 : Préparation d'une émulsion H/E stabilisée par des nanocristaux de chitine

Cette émulsion H/E peut-être préparée selon deux protocoles différents, à savoir en deux étapes ou en une seule étape.

*(a) Protocole en deux étapes*

Une première émulsion de Pickering huile dans l'eau est préparée en utilisant une phase aqueuse contenant une concentration connue de nanocristaux de chitine dans HCl 0,01 mM et NaCl 20mM.

Les émulsions ont été préparées en utilisant un ratio 20/80 huile/eau à partir d'une phase aqueuse contenant des nanoparticules à une concentration de 0,3 % en poids, par rapport au volume de la phase aqueuse (sans dilution supplémentaire).

Dans un tube, 0,2 mL d'hexadécane sont ajoutés à 0,8 mL de la suspension aqueuse ; pendant 5 secondes, le mélange est soumis à un traitement alternant 1 seconde de traitement ultrason et 2 secondes de repos.

L'émulsion de type HIPE est ensuite obtenue par l'ajout d'un volume choisi d'hexadécane au rotor-stator à 5500 tr/min, pendant 30 sec à 2 min pour 1 à 20 mL respectivement.

*(b) Protocole en une étape*

Les phases aqueuse et huile sont mises directement dans le tube.

L'émulsion de type HIPE est ensuite obtenue par mélange au rotor-stator à 5000 tr/min, pendant 30sec à 2 min.

Protocole 4 : Microscopie optique et granulométrie

Environ 20 μL de l'échantillon d'émulsion est incorporé dans 1 mL d'eau distillée.

Le produit est mélangé par vortex, puis une goutte est déposée sur une lamelle pour observation au microscope.

Le diamètre et la distribution des tailles de gouttes sont déterminés par un dispositif Malvern MasterSizer par diffraction de la lumière avec un granulomètre Malvern 2000 équipé avec un laser He-Ne (Malvern Instruments, U.K.), avec analyse par équation Fraunhofer.

Le risque d'agrégation est dans ce cas limité par l'ajout de SDS 0,1% (Sodium Dodécyl Sulfate) et HCl 0,01 mM juste avant la mesure.

Protocole 5 : Microscopie électronique à balayage (MEB)

Pour préparer l'échantillon d'émulsion en vue de son observation par microscopie électronique à balayage (MEB), 150 μL d'un mélange styrène/initiateur (ratio st : AIBN (azobisisobutyronitrile) 100:1 poids/poids) sont mélangés avec 1,0 à 1,5 mL de solution à 0,3% d'une solution échantillon d'HCl 0.01 mM dans NaCl 20mM, préalablement soumis aux ultrasons pendant 1-2 min. Le mélange est dégazé avec de l'azote pendant 10 minutes.

L'émulsion a été obtenue par traitement ultrasons pendant 30 secondes (impulsion de 2 secondes, séparées de 5 secondes).

Ce système est dégazé avec de l'azote pendant 10 minutes, et la polymérisation est mise en oeuvre à 50°C, entre 6h et 24h sans agitation.

La préparation résultante est rincée à l'HCl 0,01 mM par centrifugations répétées, puis soumise à une étape de métallisation selon les techniques conventionnelles de microscopie électronique à balayage, avant observation.

Protocole 6 : Calcul du pourcentage de phase interne

Le pourcentage de phase interne est égal au volume d'huile de l'émulsion divisé par le volume total de l'émulsion.
Ce volume peut par exemple être obtenu par prélèvement, pesée ou mesure de la hauteur d'émulsion dans un tube de diamètre connu.

Protocole 7 : Désacétylation / Déacétylation

Le procédé de désacétylation des nanocristaux de chitine est décrit dans Fan et al. Carbohydrate polymers, 2012, 79, 1046-1051.
Plus précisément, 240 mL de NaOH 40% sont ajoutés à 50 mL de suspension de nanocristaux de chitine à 1,2% contenant 0,3 g de NaBH4. Le mélange est mis 3h à 90°C sous agitation.
Au bout de 3h de lavage par centrifugations répétées, sont mis en oeuvre une dialyse contre HCl 0,01 mM puis filtration à 5$\mu$m puis 1 $\mu$m.

Protocole 8 : Préparation des mousses solides

Les émulsions dans des tubes de 10 mL sont centrifugées à 2000g pendant 2 à 5 min.
Cette étape nous permet de concentrer les émulsions et de les dégazer.
Une fois la centrifugation terminée, le fond du tube est découpé à l'aide d'un coupe tube pour évacuer toute la phase aqueuse et ne récupérer que l'émulsion concentrée.
Les émulsions sont ensuite placées au congélateur à -18°C puis lyophilisées.

Protocole 9 : Préparation de mousses sèches

Des essais de formation de mousses ont été effectués sur la base du protocole décrit dans le document Alargova et al. - « Foam superstabilization by polymer microrods », Langmuir, vol. 20(24), 2004, 10371-10374.
Ce procédé consiste en une aération par forte agitation latérale manuelle de 2mL d'une suspension dans une éprouvette de 10 mL pendant 30 secondes à 1 minute.
Le volume de mousse formé est mesuré dans l'éprouvette par une mesure de l'épaisseur de mousse à l'aide d'un pied à coulisse électronique. Toutes les mesures ont été effectuées par le même expérimentateur.

**B. Résultats**

B.1. Evolution du diamètre moyen en fonction de différents paramètres pour les émulsions de Pickering

Une méthode pour évaluer la stabilité d'une émulsion est de mesurer la taille des gouttes pour vérifier s'il y a coalescence.
Le diamètre moyen a donc été mesuré par granulométrie (Protocole 4).

*(a) Concentration en nanocristaux de chitine*

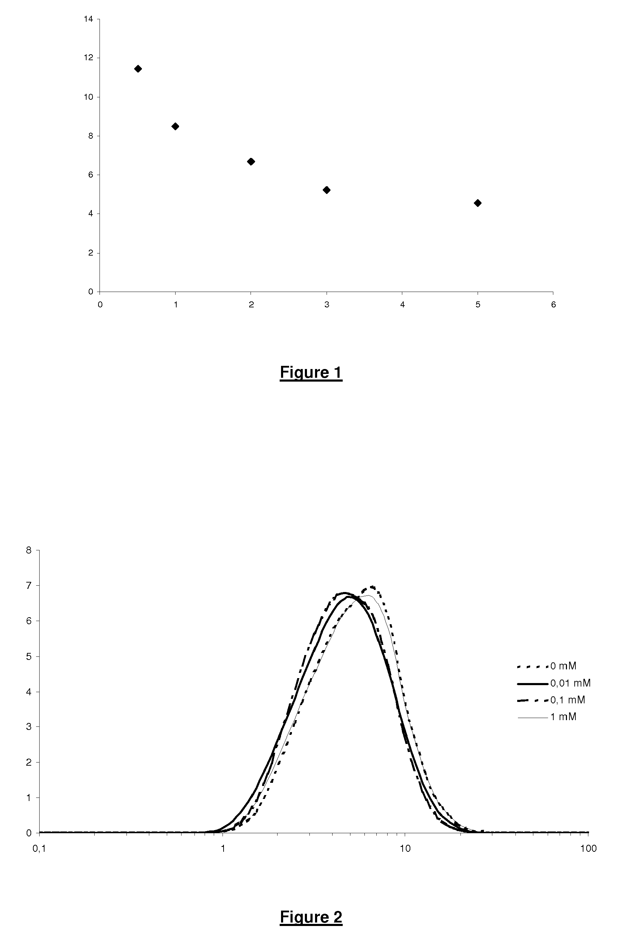

Les résultats obtenus sont présentés dans le tableau 1 ci-dessous et dans la figure 1.

**Tableau 1 :** Evolution du diamètre moyen en fonction de la concentration en nanocristaux de chitine, dans une solution à NaCl 20mM et HCl 0,01 mM

| Concentration en nanocristaux (g/L) | D3,2 ($\mu$m) | D4,3 ($\mu$m) | Polydispersité |
|---|---|---|---|
| 0,5 | 11,45 $\pm$ 0.64 | 13,25 $\pm$ 0.49 | 1,51 |
| 1 | 8,51 $\pm$ 0.21 | 10,06 $\pm$ 0.57 | 1,58 |
| 2 | 6,67 $\pm$ 0.12 | 8,76 $\pm$ 0.21 | 1,31 |
| 3 | 5,21 $\pm$ 0.16 | 6,96 $\pm$ 0.36 | 1,34 |
| 5 | 4,54 $\pm$ 0.07 | 6,19 $\pm$ 0.10 | 1,36 |

Au regard de ces résultats, il y a une diminution continue du diamètre avec l'augmentation du nombre de nanocristaux de chitine introduits, puis une stabilisation.
Une valeur préférée pour la concentration de nanocristaux de chitine serait de 3g/L.

*(b) Variation du pH*

Les résultats obtenus sont présentés dans le tableau 2 ci-dessous et dans la figure 2.

**Tableau 2 :** Evolution du diamètre moyen ($\mu$m) en fonction du pH ajusté par la concentration en HCl (pour des émulsions à 3g/L de nanocristaux de chitine dans une solution à NaCl 20mM)

| Concentration en HCl (mM) | D3,2 ($\mu$m) | D4,3 ($\mu$m) | Polydispersité |
|---|---|---|---|
| 0 | 5,83 $\pm$ 0.17 | 7,61 $\pm$ 0,18 | 1,31 |
| 0.01 | 5,21 $\pm$ 0.16 | 6,96 $\pm$ 0,36 | 1,34 |
| 0.1 | 5,10 $\pm$ 0.15 | 6,58 $\pm$ 0,22 | 1,29 |
| 1 | 5,78 $\pm$ 0.22 | 7,60 $\pm$ 0,27 | 1,32 |

Ces résultats mettent en évidence qu'il n'y a pas d'influence du pH sur la préparation d'émulsions de Pickering à partir de nanocristaux de chitine.

Une valeur préférée pour le pH serait de 0,01 à 0,1 mM de HCl.

*(c) Variation de la salinité*

Les résultats obtenus sont présentés dans le tableau 3 ci-dessous.

**Tableau 3 :** Evolution du diamètre moyen ($\mu$m) en fonction de la salinité (mM) par variation de la concentration en NaCl (pour des émulsions à 3g/L de nanocristaux de chitine et une concentration en HCl à 0,01 mM)

| Concentration en NaCl (mM) | D3,2 ($\mu$m) | D4,3 ($\mu$m) | Polydispersité |
|---|---|---|---|
| 0 | 8,32 $\pm$ 0.32 | 13,00 $\pm$ 0,13 | 1,56 |
| 2 | 5,84 $\pm$ 0.16 | 7,53 $\pm$ 0,38 | 1,29 |
| 5 | 5,85 $\pm$ 0.08 | 7,32 $\pm$ 0,34 | 1,25 |
| 10 | 6,23 $\pm$ 0.10 | 8,03 $\pm$ 0,16 | 1,29 |
| 20 | 6,43 $\pm$ 0.21 | 8,43 $\pm$ 0,32 | 1,31 |
| 50 | 6,39 $\pm$ 0.20 | 8,25 $\pm$ 0,35 | 1,29 |
| 100 | 5,35 $\pm$ 0.24 | 7,02 $\pm$ 0,37 | 1,31 |

En absence de force ionique, la production d'émulsion est possible mais s'avère moins maîtrisée et la distribution de taille des gouttes plus polydisperse.

A partir d'une concentration de 2mM en NaCl dans nos conditions, il n'y a pas d'influence de la force ionique.

Une valeur préférée serait de 20 ou 50 mM de NaCl, selon la densité de charge de surface et la concentration.

*(d) Stabilité au stockage à différentes températures.*

Les échantillons dans nos conditions standards (3g/L de nanocristaux de chitine dans NaCl 20mM et HCl 0.01 mM) ont été stockés pendant une semaine à différentes températures.

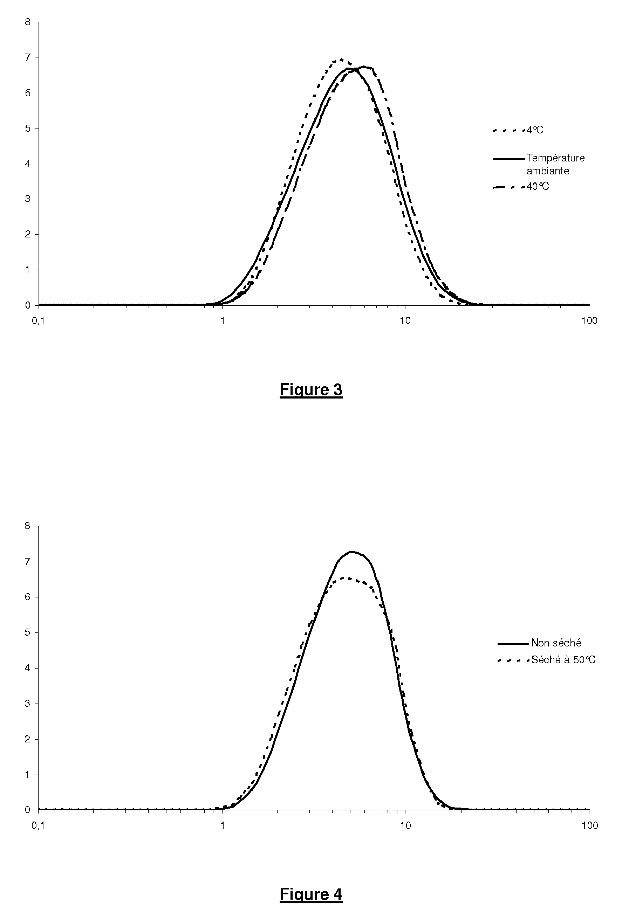

Les résultats obtenus sont présentés dans le tableau 4 ci-dessous et dans la figure 3.

**Tableau 4 :** Evolution du diamètre moyen (um) en fonction de la température de stockage (°C)

| Température de stockage | D3,2 ($\mu$m) | D4,3 ($\mu$m) | Polydispersité |
|---|---|---|---|
| 25°C | 5,21 $\pm$0.16 | 6,96 $\pm$ 0.36 | 1,34 |
| 4°C | 4,94 $\pm$ 0.18 | 6,36 $\pm$ 0.23 | 1,29 |
| 40°C | 5,60 $\pm$ 0.15 | 7,10 $\pm$ 0.08 | 1,27 |

Après ces traitements thermiques, les émulsions étaient visiblement inchangées.

La valeur à 25°C sert de valeur de référence et l'absence de coalescence montre la stabilité des émulsions dans ces conditions.

B.2. Stabilité au séchage en vue de l'application comme émulsion sèche

Un échantillon d'émulsion à 5 g/L de nanocristaux de chitine dans NaCl 50mM et HCl 0,01mM a été maintenu dans un tube non bouché à 50°C pendant 8h puis à 25°C pendant 5 jours (temps nécessaire pour sécher de façon homogène).

Il a ensuite été redispersé dans HCl 0,01 mM et passé au granulomètre.

Les résultats obtenus sont présentés dans le tableau 5 ci-dessous et dans la figure 4 annexée.

**Tableau 5 :** Evolution du diamètre moyen ($\mu$m) avant et après séchage

|  | D3,2 ($\mu$m) | D4,3 ($\mu$m) | Polydispersité |
|---|---|---|---|
| Non séché | 5,07 $\pm$ 0.04 | 6,33 $\pm$ 0.07 | 1,25 |
| Séché à 50°C puis 25°C | 5,05 $\pm$ 0.13 | 6,50 $\pm$ 0.35 | 1,29 |

Le diamètre des gouttes est identique, montrant la capacité de former des émulsions sèches.

B.3. Evolution du pourcentage de phase interne en fonction de différents paramètres pour les HIPEs produites selon 2 protocoles (« direct » et « deux étapes »)

Les émulsions sont préparées selon les protocoles ci-dessus et, après un temps d'attente de 24h, les volumes sont mesurés pour calculer les pourcentages de phase interne (Protocole 6).

Enfin les tubes sont penchés, voir retournés, pour donner une indication sur la texture : S : solide, C : coulant, L : liquide.

Les résultats obtenus sont développés dans les tableaux 6 à 9 ci-dessous.

**Tableau 6 :** Evolution dans nos conditions de référence : nanocristaux de chitine à 3g/L dans la phase aqueuse à NaCl 20mM et HCl 0,01 mM

| V huile ajoutée (mL) | 0,5 | 1 | 1.5 | 2.5 | 4 | 9 | 15 | 20 |
|---|---|---|---|---|---|---|---|---|
| % phase interne «deux étapes» | 70,2 $\pm$ 1,3 | 76,8 $\pm$ 0,7 | 74,3 $\pm$ 3,1 | 77,0 $\pm$ 0,0 | 83,6 $\pm$ 0,1 | 91,6 $\pm$ 0,1 | 94,4 $\pm$ 0,1 | 94,9 $\pm$ 0,3 |
|  | S | S | S | S | S | S | S | S/C |
| % phase interne en direct | 64,2 $\pm$ 5,1 | 64,7 $\pm$ 0,6 | 68,0 $\pm$ 0,0 | 77,1 $\pm$ 0,0 | 83,9 $\pm$ 0,0 | 91,6 $\pm$ 0,2 | 94,9 $\pm$ 0,0 | 84,8 |
|  | S | S | S | S | S | S | S | S |

**Tableau 7 :** Evolution en fonction du pH, à plus forte acidité : nanocristaux de chitine à 3g/L dans la phase aqueuse NaCl 20mM et HCl 1 mM.

| V huile ajoutée (mL) | 0,5 | 1 | 1.5 | 2.5 | 4 | 9 | 15 | 20 |
|---|---|---|---|---|---|---|---|---|
| % phase interne «deux étapes» | 72,6 $\pm$ 4,3 | 74,2 $\pm$ 0,5 | 75,1 $\pm$ 1,1 | 76,9 $\pm$ 0,1 | 83,6 $\pm$ 0,0 | 91,3 $\pm$ 0,3 | 94,2 $\pm$ 0,1 | 96,1 $\pm$ 0,0 |
|  | S | S | S | S | S | S | S | S/C |
| % phase interne en direct | 69,8 $\pm$ 1,9 | 67,8 $\pm$ 0,9 | 67,7 $\pm$ 0,1 | 76,7 $\pm$ 0,1 | 83,6 $\pm$ 0,0 | 91,5 $\pm$ 0,0 | 94,5 $\pm$ 0,4 | 96,0 |
|  | S | S | S | S | S | S | S | S |

**Tableau 8 :** Evolution en fonction de la salinité, en absence de NaCl : nanocristaux de chitine à 3g/L dans la phase aaueuse HCl 0,01 mM.

| V huile ajoutée (mL) | 0,5 | 1 | 1.5 | 2.5 | 4 | 9 | 15 | 20 |
|---|---|---|---|---|---|---|---|---|

(suite)

| % phase interne «deux étapes» | 70,3 ± 0,2 | 74,6 ± 3,3 | 77,7 ± 3,3 | 77,6 ± 3,1 | 83,0 ± 0,0 | 91,7 ± 0,1 | 94,6 ± 0,0 | 95,6 ± 0,8 |
|---|---|---|---|---|---|---|---|---|
| | C | C | C | S | S | S | S | S/C |
| % phase interne en direct | 72,3 ± 3,4 | 69,7 ± 1,1 | 71,8 ± 2,0 | 83,7 ± 4,6 | 83,5 ± 0,2 | 91,9 ± 0,3 | 94,6 ± 0,1 | 96,0 ± 0,1 |
| | L | L | L | C | S | S | S | S/C |

**Tableau 9 :** Evolution en fonction de la salinité, à haute concentration en NaCl : nanocristaux de chitine à 3g/L dans la phase aqueuse avec NaCl 100mM et HCl 0.01 mM.

| V huile ajoutée (mL) | 0,5 | 1 | 1.5 | 2.5 | 4 | 9 | 15 | 20 |
|---|---|---|---|---|---|---|---|---|
| % phase interne «deux étapes» | 67,1 ± 5,1 | 70,2 ± 1,2 | 69,8 ± 1,8 | 76,9 ± 0,1 | 82,2 ± 2,1 | 91,3 ± 0,1 | 94,3 ± 0,3 | 93,4 ± 0,2 |
| | S | S | S | S | S | S | S | S/C |
| % phase interne en direct | 40,7 ± 0,3 | 60,0 ± 0,0 | 68,0 ± 0,0 | 77,1 ± 0,1 | 83,6 ± 0,0 | 91,7 ± 0,0 | 94,8 ± 0,0 | 95,9 ± 0,2 |
| | C | S | S | S | S | S | S | S/C |

Si des différences sont observées pour les premiers points d'émulsions moins stables et moins structurées en absence de sel (gel coulant/gel solide), au-delà de environ 74% de phase interne, toutes les émulsions présentent un pourcentage de phase interne identique, indépendamment de la méthode et des paramètres testés.

Dans la plupart des cas, un gel solide est obtenu.

Seule l'absence de sel conduit à des émulsions moins structurées, mais il est toujours possible de former des HIPEs. A forte salinité, le gel étant très structuré, il est plus difficile de le mettre en oeuvre.

La voie « directe » intègre la totalité de l'eau introduite, alors que la voie en « deux étapes » a tendance à exclure de l'eau, augmentant ainsi le pourcentage de phase interne pour les MIPE.

Au-delà d'environ 74% de phase interne, les pourcentages de phase interne sont identiques pour les deux procédés.

On notera cependant une différence de texture notable entre les deux méthodes. La structuration du gel selon le mode de préparation semble donc différente.

Si l'on veut un gel plus structuré (gel solide) et un pourcentage de phase interne supérieur, il est préférable de le faire selon la voie en deux étapes.

Par ailleurs, des résultats (non présentés) mettent en évidence qu'il est possible de préparer des MIPEs et des HIPEs à partir de nanocristaux de chitine, après 1 et 2 heures de déacétylation selon le protocole 7.

B.4. Préparation de mousses solides à partir d'émulsion de Pickering ou de MIPE

Les émulsions préparées à base de nanocristaux de chitine et de cyclohexane sont congelées puis lyophilisées (Protocole 8).

Ce protocole permet d'obtenir des mousses alvéolaires dont les tailles de cellules sont maîtrisables selon le volume de phase interne (cyclohexane) ajouté à une émulsion de Pickering.

Des images MEB mettent en évidence ces structures alvéolaires. Ceci illustre la grande stabilité des nanocristaux de chitine à l'interface, qui permet de conserver la structure pour une très haute porosité et des murs extrêmement fins, c'est-à-dire de l'ordre de 10 à 50 nanomètres.

Ces résultats sont liés aux propriétés des émulsions de Pickering car l'adsorption irréversible à l'interface et la grande stabilité de ces émulsions permet le maintien de la structure à la congélation.

Pour une voie en deux étapes (résultats non présentés), la structure de la mousse est analogue.

B.5. Combinaison de nanocristaux de polysaccharide

Des émulsions mixtes de nanocristaux chitine / cellulose ont été préparées selon le protocole 3(a) en « deux étapes » ci-dessus, de sorte à obtenir une combinaison de nanocristaux de chitine à 1,5g/L et de nanocristaux de cellulose à 1,5g/L dans la phase aqueuse contenant du NaCl 20mM et du HCl 0,01 mM.

Après un temps d'attente, les volumes sont mesurés pour calculer les pourcentages de phase interne (Protocole 6).

Enfin les tubes sont penchés, voire retournés, pour donner une indication sur la texture : S : solide, C : coulant, L : liquide.

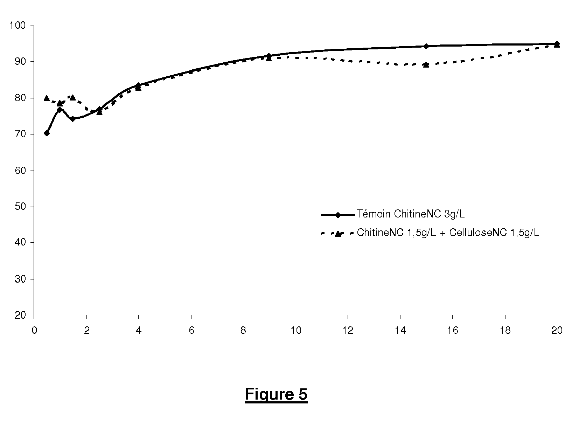

Les résultats obtenus sont développés dans le tableau 10 ci-dessous et dans la figure 5 annexée.

**Tableau 10 :** Evolution du pourcentage de phase interne pour une suspension mixte chitine / cellulose

| V huile ajoutée (mL) | 0,5 | 1 | 1,5 | 2,5 | 4 | 9 | 15 | 20 |
|---|---|---|---|---|---|---|---|---|
| % phase interne en « deux étapes » | 79,9 ± 5,4 | 78,6 ± 1,6 | 80,2 ± 2,1 | 76,2 ± 0,1 | 82,9 ± 0,1 | 91,0 ± 0,2 | 89,3 ± 7,1 | 94,9 ± 0,5 |
| | S | S | S | S | S | S | S | C |

Les propriétés restent inchangées, seules la nature de l'interface étant modifiée.

Une suspension comportant 50% de nanocristaux de chitine et 50% de nanocristaux de cellulose conduit à des MIPEs et HIPEs équivalents en texture et en pourcentage de phase interne à une suspension comprenant 100% de nanocristaux de chitine, mais dont les charges de surfaces seront à la fois positives et négatives ce qui peut être intéressant pour certaines applications.

B.6. Formation d'une mousse sèche stable

Des essais de formation de mousses sèches ont été mis en oeuvre selon le protocole 9 ci-dessus.

Les différents paramètres suivants ont été étudiés :

- une concentration en nanocristaux de chitine de 2g/L à 13g/L,
- une force ionique entre 0 et 5mM de NaCl,
- un pH à 0,01 mM HCL (pH5), 0,1 mM HCl (pH4) et 1 mM HCl (pH3).

*Concentration de chitine*

Les résultats obtenus sont présentés dans le tableau 11 ci-dessous, en fraction volumique de mousse par rapport au volume total d'émulsion.

**Tableau 11 :** Comparaison des fraction de mousse selon les concentrations en nanocristaux de chitine et stabilité dans le temps (0,01mM HCl, 2mM NaCl)

| | | Concentration en nanocristaux de chitine (g/L) | | | |
|---|---|---|---|---|---|
| | | 2 | 6 | 10 | 13 |
| Temps | 0 | 25% | 41% | 47% | 89% |
| | 2 min | 21% | 33% | 41% | 78% |
| | 20 min | 10% | 30% | 40% | 76% |
| | 12 h | 10% | 29% | 40% | 75% |
| | 2 jours | 8% | 23% | 28% | 61% |
| | 5 jours | 4% | 22% | 25% | 55% |

La formation de mousse est effectivement observée à partir d'une concentration de 6g/L en nanocristaux de chitine. Pour des concentrations inférieures à 6g/L, la mousse est très faible et instable.

*Force ionique et pH*

Les résultats obtenus sont présentés dans le tableau 12 ci-dessous

**Tableau 12 :** Comparaison des évolutions des fractions volumiques de mousse en pourcentage, selon les conditions de force ionique et pH à 13g/L de nanocristaux de chitine et T=20 min

| | | NaCl (mM) | | | |
|---|---|---|---|---|---|
| | | 0 | 1 | 2 | 5 |
| HCl (mM) | 0,01 | 67% | 79% | 76% | 70% |
| | 0,1 | 32% | 41% | 61% | 40% |
| | 1 | 0,03% | 0,7% | 28% | 25% |

Comme le montrent les évolutions de volume de mousse mesuré, l'absence de force ionique (NaCl à 0 mM) ne permet par la formation de mousse pour des pH inférieur à 4. De même, l'augmentation de la force ionique au-delà de 10mM tend à une déstabilisation de la mousse quel que soit le pH.

Par ailleurs, l'augmentation du pH de 3 à 5 favorise la stabilisation de la mousse.

Il a également été observé qu'une mousse d'origine à pH5 disparaît à pH2 (par ajout de $20\mu L$ HCl à 1 M) ; seule subsiste une couronne. Une mousse se reforme lors du retour à pH5 (par ajout de $20\mu L$ NaOH à 1 M).

Ces résultats montrent le caractère réversible de la mousse en fonction du pH.

*Etude de la stabilité des mousses dans le temps*

Une solution de chitine de 2g/L à 13g/L (0,01 mM HCl et 5mM NaCl) est soumise à une agitation horizontale selon le protocole 9 précité.

Les résultats obtenus sont présentés dans le tableau 11 ci-dessus.

Il est observé une très bonne stabilité dans le temps.

**Revendications**

1. Composition comprenant une phase interne dispersée dans une phase continue hydrophile, possédant un pourcentage de phase interne supérieur à 50% en volume, **caractérisée en ce que** ladite composition contient des nanocristaux d'un polysaccharide autre que la cellulose qui sont localisés à l'interface entre ladite phase interne et ladite phase continue hydrophile.

2. Composition selon la revendication 1, **caractérisée en ce que** les nanocristaux d'un polysaccharide, autre que la cellulose, sont choisis parmi les nanocristaux qui sont chargés positivement.

3. Composition selon la revendication 2, **caractérisée en ce que** les nanocristaux de polysaccharide sont choisis parmi :

    (i) exclusivement des nanocristaux de polysaccharide chargés positivement, à l'exclusion de nanocristaux de cellulose, ou
    (ii) des nanocristaux de polysaccharide chargés positivement, autre que la cellulose, mélangés avec des nanocristaux issus d'au moins un polysaccharide chargés négativement.

4. Composition selon l'une quelconque des revendications 2 ou 3, **caractérisée en ce que** les nanocristaux d'un polysaccharide, autre que la cellulose, sont choisis parmi les nanocristaux de chitine.

5. - Composition selon l'une quelconque des revendications 1 à 4, **caractérisée en ce qu'**elle consiste en une émulsion ou en une mousse.

6. - Composition selon l'une quelconque des revendications 1 à 5, **caractérisée en ce que** ladite composition consiste en une émulsion (i) du type à haute phase interne ou HIPE, possédant un pourcentage de phase interne supérieur à 75% en volume, ou (ii) du type à moyenne phase interne ou MIPE, possédant un pourcentage de phase interne compris entre 50% et à 75% en volume.

7. Procédé d'obtention d'une composition comprenant une phase interne dispersée dans une phase continue hydrophile, possédant un pourcentage de phase interne supérieur à 50% en volume, lequel procédé est **caractérisé en ce qu'**il comprend les opérations suivantes :

    a) une opération d'incorporation de nanocristaux d'un polysaccharide autre que la cellulose, dans une phase hydrophile,
    b) une opération de fourniture de ladite phase hydrophile contenant les nanocristaux de polysaccharide et d'une phase destinée à constituer ladite phase interne,
    c) une opération de formation de ladite composition par dispersion de ladite phase interne dans ladite phase hydrophile.

8. - Procédé selon la revendication 7, **caractérisé en ce que** les nanocristaux de polysaccharide incorporés à l'opération a) sont choisis parmi les nanocristaux qui sont chargés positivement.

9. - Procédé selon la revendication 8, **caractérisé en ce que** les nanocristaux de polysaccharide incorporés à l'opération a) sont choisis parmi :

    (i) exclusivement des nanocristaux de polysaccharide chargés positivement, à l'exclusion de nanocristaux de cellulose, ou
    (ii) des nanocristaux de polysaccharide chargés positivement, autre que la cellulose, mélangés avec des nanocristaux issus d'au moins un polysaccharide chargés négativement.

10. - Procédé selon l'une quelconque des revendications 8 ou 9, **caractérisé en ce que** les nanocristaux de polysaccharide incorporés à l'opération a) sont choisis parmi les nanocristaux de chitine.

11. - Procédé selon l'une quelconque des revendications 7 à 10, **caractérisé en ce que**, à l'issue de l'opération c), la composition obtenue consiste en une émulsion ou en une mousse.

**12.** - Procédé selon l'une quelconque des revendications 7 à 11, **caractérisé en ce que** la composition est une composition d'émulsion comprenant une phase interne hydrophobe et une phase continue hydrophile, et **en ce que** l'opération c) de formation de l'émulsion comprend :

c.1) une étape d'obtention d'une émulsion huile-dans-l'eau, intermédiaire, ayant un rapport volumique phase interne hydrophobe / phase continue hydrophile d'au moins 5/95,
c.2) une étape d'obtention de la composition émulsion possédant un pourcentage de phase interne supérieur à 50% en volume, comprenant :

c.2.1) une étape d'ajout d'un volume de phase hydrophobe à l'émulsion intermédiaire obtenue à l'étape c.1), et agitation du mélange ainsi obtenu, et/ou
c.2.2) une étape de concentration de l'émulsion intermédiaire obtenue à l'étape c.1), par retrait d'au moins une partie de ladite phase hydrophile.

**13.** Procédé selon la revendication 12, **caractérisé en ce qu'**à l'étape c.1), l'émulsion huile-dans-eau intermédiaire a un pourcentage de phase interne en volume inférieur ou égal à 50% en volume.

**14.** - Procédé selon l'une quelconque des revendications 7 à 11, **caractérisé en ce que** la composition est une composition d'émulsion comprenant une phase interne hydrophobe et une phase continue hydrophile, et **en ce que** l'opération c) de formation de l'émulsion consiste à mélanger la phase hydrophile contenant les nanocristaux de polysaccharide et la phase interne hydrophobe, pour l'obtention directement de ladite émulsion possédant un pourcentage de phase interne supérieur à 50% en volume.

**15.** - Procédé selon l'une quelconque des revendications 7 à 14, **caractérisé en ce que**, à l'issue de l'étape c), la composition d'émulsion formée est (i) du type à haute phase interne ou HIPE, possédant un pourcentage de phase interne supérieur à 75% en volume, ou (ii) du type à moyenne phase interne ou MIPE, possédant un pourcentage de phase interne compris entre 50% et 75% en volume.

**Patentansprüche**

**1.** Zusammensetzung, umfassend eine innere Phase, die in einer kontinuierlichen hydrophilen Phase dispergiert ist, die einen Prozentanteil an innerer Phase von mehr als 50 Vol.-% aufweist, **dadurch gekennzeichnet, dass** die Zusammensetzung Nanokristalle eines Polysaccharids, bei dem es sich nicht um Cellulose handelt, enthält, die an der Grenzfläche zwischen der inneren Phase und der kontinuierlichen hydrophilen Phase liegen.

**2.** Zusammensetzung nach Anspruch 1, **dadurch gekennzeichnet, dass** die Nanokristalle eines Polysaccharids, bei dem es sich nicht um Cellulose handelt, ausgewählt sind aus Nanokristallen, die positiv geladen sind.

**3.** Zusammensetzung nach Anspruch 2, **dadurch gekennzeichnet, dass** die Polysaccharidnanokristalle ausgewählt sind aus:

(i) ausschließlich positiv geladenen Polysaccharidnanokristallen, wobei Cellulosenanokristalle ausgeschlossen sind, oder
(ii) positiv geladenen Polysaccharidnanokristallen, bei denen es sich nicht um Cellulose handelt, gemischt mit Nanokristallen, die aus mindestens einem negativ geladenen Polysaccharid stammen.

**4.** Zusammensetzung nach einem der Ansprüche 2 oder 3, **dadurch gekennzeichnet, dass** die Nanokristalle eines Polysaccharids, bei dem es sich nicht um Cellulose handelt, ausgewählt sind aus Chitinnanokristallen.

**5.** Zusammensetzung nach einem der Ansprüche 1 bis 4, **dadurch gekennzeichnet, dass** sie aus einer Emulsion oder einem Schaum besteht.

**6.** Zusammensetzung nach einem der Ansprüche 1 bis 5, **dadurch gekennzeichnet, dass** die Zusammensetzung aus einer Emulsion (i) des Typs mit hohem Anteil an innerer Phase oder HIPE, die einen Prozentanteil an innerer Phase von mehr als 75 Vol.-% aufweist, oder (ii) des Typs mit einem mittleren Anteil an innerer Phase oder MIPE, die einen Prozentanteil an innerer Phase im Bereich zwischen 50 Vol.-% und 75 Vol.-% aufweist, besteht.

7.  Verfahren zum Erhalt einer Zusammensetzung, umfassend eine innere Phase, die in einer kontinuierlichen hydrophilen Phase dispergiert ist, die einen Prozentanteil an innerer Phase von mehr als 50 Vol.-% aufweist, wobei das Verfahren **dadurch gekennzeichnet ist, dass** es die folgenden Vorgänge umfasst:

    a) einen Vorgang zum Integrieren von Nanokristallen eines Polysaccharids, bei dem es sich nicht um Cellulose handelt, in eine hydrophile Phase,
    b) einen Vorgang zum Bereitstellen der hydrophilen Phase, die die Polysaccharidnanokristalle enthält, und einer Phase, die dazu bestimmt ist, die innere Phase zu bilden,
    c) einen Vorgang zum Bilden der Zusammensetzung durch Dispergieren der inneren Phase in der hydrophilen Phase.

8.  Verfahren nach Anspruch 7, **dadurch gekennzeichnet, dass** die in Vorgang a) integrierten Polysaccharidnanokristalle ausgewählt sind aus Nanokristallen, die positiv geladen sind.

9.  Verfahren nach Anspruch 8, **dadurch gekennzeichnet, dass** die in Vorgang a) integrierten Polysaccharidnanokristalle ausgewählt sind aus:

    (i) ausschließlich positiv geladenen Polysaccharidnanokristallen, wobei Cellulosenanokristalle ausgeschlossen sind, oder
    (ii) positiv geladenen Polysaccharidnanokristallen, bei denen es sich nicht um Cellulose handelt, gemischt mit Nanokristallen, die aus mindestens einem negativ geladenen Polysaccharid stammen.

10. Verfahren nach einem der Ansprüche 8 oder 9, **dadurch gekennzeichnet, dass** die in Vorgang a) integrierten Polysaccharidnanokristalle ausgewählt sind aus Chitinnanokristallen.

11. Verfahren nach einem der Ansprüche 7 bis 10, **dadurch gekennzeichnet, dass** am Ende von Vorgang c) die erhaltene Zusammensetzung aus einer Emulsion oder einem Schaum besteht.

12. Verfahren nach einem der Ansprüche 7 bis 11, **dadurch gekennzeichnet, dass** die Zusammensetzung eine Emulsionszusammensetzung ist, die eine innere hydrophobe Phase und eine kontinuierliche hydrophile Phase umfasst, und dadurch, dass der Vorgang c) zum Bilden der Emulsion Folgendes umfasst:

    c.1) einen Schritt zum Erhalt einer Öl-in-Wasser-Zwischenemulsion, die ein Volumenverhältnis von innerer hydrophober Phase zu kontinuierlicher hydrophiler Phase von mindestens 5/95 aufweist,
    c.2) einen Schritt zum Erhalt der Emulsionszusammensetzung, die einen Prozentanteil an innerer Phase von mehr als 50 Vol.-% ausweist, umfassend:

        c.2.1) einen Schritt zum Zugeben eines Volumens der hydrophoben Phase zu der in Schritt c.1) erhaltenen Zwischenemulsion und Rühren des auf diese Weise erhaltenen Gemischs und/oder
        c.2.2) einen Schritt zur Konzentration der in Schritt c.1) erhaltenen Zwischenemulsion durch Entfernen mindestens eines Teils der hydrophilen Phase.

13. Verfahren nach Anspruch 12, **dadurch gekennzeichnet, dass** in Schritt c.1) die Öl-in-Wasser-Zwischenemulsion einen Volumenprozentanteil an innerer Phase von kleiner oder gleich 50 Vol.-% hat.

14. Verfahren nach einem der Ansprüche 7 bis 11, **dadurch gekennzeichnet, dass** die Zusammensetzung eine Emulsionszusammensetzung ist, die eine innere hydrophobe Phase und eine kontinuierliche hydrophile Phase umfasst, und dadurch, dass der Vorgang c) zum Bilden der Emulsion darin besteht, die hydrophile Phase, die die Polysaccharidnanokristalle enthält, und die innere hydrophobe Phase zu mischen, um die Emulsion, die einen Prozentanteil an innerer Phase von mehr als 50 Vol.-% aufweist, direkt zu erhalten.

15. Verfahren nach einem der Ansprüche 7 bis 14, **dadurch gekennzeichnet, dass** am Ende von Schritt c) die gebildete Emulsionszusammensetzung (i) vom Typ mit hohem Anteil an innerer Phase oder HIPE, die einen Prozentanteil an innerer Phase von mehr als 75 Vol.-% aufweist, oder (ii) vom Typ mit einem mittleren Anteil an innerer Phase oder MIPE, die einen Prozentanteil an innerer Phase im Bereich zwischen 50 Vol.-% und 75 Vol.-% aufweist, ist.

**Claims**

1. Composition comprising an internal phase dispersed in a hydrophilic continuous phase, the percentage of the internal phase being higher than 50% in volume, **characterized in that** said composition contains nanocrystals of a polysaccharide other than cellulose, which are located at the interface between said internal phase and said hydrophilic continuous phase.

2. Composition according to claim 1, **characterized in that** the nanocrystals of a polysaccharide other than cellulose are selected from nanocrystals which are positively charged.

3. Composition according to claim 2, **characterized in that** the nanocrystals of polysaccharide are selected from:

   (i) exclusively positively charged polysaccharide nanocrystals, excluding cellulose nanocrystals, or
   (ii) positively charged polysaccharide nanocrystals, other than cellulose, mixed with nanocrystals derived from at least one negatively charged polysaccharide.

4. Composition according to any one of claims 2 or 3, **characterized in that** the nanocrystals of a polysaccharide other than cellulose are selected from chitin nanocrystals.

5. Composition according to any one of claims 1 to 4, **characterized in that** it consists in an emulsion or a foam.

6. Composition according to any one of claims 1 to 5, **characterized in that** said composition consists in an emulsion (i) of the high internal phase or HIPE type, having a percentage of internal phase higher than 75% in volume, or (ii) of the medium internal phase or MIPE type, having a percentage of internal phase ranging between 50% and 75% in volume.

7. Process for obtaining a composition comprising an internal phase dispersed in a hydrophilic continuous phase, the percentage of the internal phase being higher than 50% in volume, which process is **characterized in that** it comprises the following operations:

   a) an operation of incorporating nanocrystals of a polysaccharide other than cellulose, in a hydrophilic phase,
   (b) an operation of providing said hydrophilic phase containing the polysaccharide nanocrystals and a phase for constituting said internal phase,
   c) an operation of forming said composition by dispersing said internal phase in said hydrophilic phase.

8. Process according to claim 7, **characterized in that** the polysaccharide nanocrystals incorporated in operation a) are selected from nanocrystals which are positively charged.

9. Process according to claim 8, **characterized in that** the polysaccharide nanocrystals incorporated in operation a) are selected from:

   (i) exclusively positively charged polysaccharide nanocrystals, excluding cellulose nanocrystals, or
   (ii) positively charged polysaccharide nanocrystals, other than cellulose, mixed with nanocrystals derived from at least one negatively charged polysaccharide.

10. Process according to any one of claims 8 or 9, **characterized in that** the polysaccharide nanocrystals incorporated in operation a) are selected from chitin nanocrystals.

11. Process according to any one of claims 7 to 10, **characterized in that**, at the end of the operation c), the obtained composition consists in an emulsion or a foam.

12. Process according to any one of claims 7 to 11, **characterized in that** the composition is an emulsion composition comprising a hydrophobic internal phase and a hydrophilic continuous phase, and **in that** operation c) of forming the emulsion comprises:

   c.1) a step of obtaining an intermediate oil-in-water emulsion having a volume ratio hydrophobic internal phase/hydrophilic continuous phase of at least 5/95,
   c.2) a step of obtaining the emulsion composition having a percentage of internal phase higher than 50% in volume, comprising:

c.2.1) a step of adding a hydrophobic phase volume to the intermediate emulsion obtained in step c.1), and stirring the mixture thus obtained, and/or

c.2.2) a step of concentrating the intermediate emulsion obtained in step c.1) by removing at least a part of said hydrophilic phase.

13. Process according to claim 12, **characterized in that** in step c.1), the intermediate oil-in-water emulsion has a percentage of internal phase that is equal to or less than 50% in volume.

14. Process according to any one of claims 7 to 11, **characterized in that** the composition is an emulsion composition comprising a hydrophobic internal phase and a hydrophilic continuous phase, and **in that** operation c) of forming the emulsion consists in mixing the hydrophilic phase containing the polysaccharide nanocrystal and the hydrophobic internal phase to directly obtain said emulsion having a percentage of internal phase higher than 50% in volume.

15. Process according to any one of claims 7 to 14, **characterized in that**, at the end of step c), the emulsion composition formed is (i) of the high internal phase or HIPE type, having a percentage of internal phase higher than 75% in volume, or (ii) of the medium internal phase or MIPE type, having a percentage of internal phase ranging between 50% and 75% in volume.

**Figure 1**

**Figure 2**

**Figure 3**

**Figure 4**

**Figure 5**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010058148 A
- WO 2009013500 A
- US 6218440 B
- US 4472086 A

**Littérature non-brevet citée dans la description**

- **TZOUMAKI et al.** Oil-in-water emulsions stabilized by chitin nanocrystal particles. *FOOD HYDROCOLLOIDS,* 2011, vol. 25, 1521-1529
- **L. CANTAT et al.** Les mousses : structure et dynamique. 2010, 278
- **L. CANTAT et al.** Les mousses : Structure et Dynamique. 2010, 278
- **SAMIR et al.** *Biomacromolecules,* 2005, vol. 6, 612-626
- **ELAZZOUZI-HAFRAOUI et al.** *Biomacromolecules,* 2008, vol. 9 (1), 57-65
- **REVOL JF ; MARCHESSAULT RH.** *Int. J. Biol. Macromol.,* 1993, vol. 15, 325
- **MURRAY S.B. ; NEVILLE A.C.** *Int J. Biol. Macromol.,* 1997, vol. 20, 123-130
- **NAIR ; DUFRESNE.** *Biomacromolecules,* 2003
- **BELAMIE et al.** *J. Phys. Chem.,* 2004
- **PHONGGYING et al.** *Polymer,* 2007
- **M. RINAUDO.** *Progress in Polymer Science,* 2006, vol. 31, 603-632
- **Y. M. FAN ; T. SAITO ; A. ISOGAI.** *Carbohydrate Polymers,* 2010, vol. 79, 1046-1051
- **LUCASSEN, J.** Anionic Surfactants - Physical Chemistry of Surfactant Action NY. Marcel Dekker, 1981
- **ALARGOVA et al.** Foam superstabilization by polymer microrods. *Langmuir,* 2004, vol. 20 (24), 10371-10374
- **BLANCO E. et al.** Stability and Viscoelasticity of Magneto-Pickering Foams. *Langmuir,* 2013, vol. 29, 10019-10027
- **A. OSORIO-MADRAZOA et al.** *Carbohydrate Polymers,* 2011, vol. 83 (4), 1730-1739
- **TZOUMAKI et al.** Oil-in water emulsions stabilized by chitin nanocrystal particles. *Food Hydrocolloids,* 2011, vol. 25, 1521-1529
- **PAUL BECHER.** Emulsions: Theory and Practice. Oxford University Press, 2001
- **CAMERON NR ; SHERRINGTON DC.** High internal phase emulsions (HIPEs) - Structure, properties and use in polymer preparation. *BIOPOLYMERS LIQUID CRYSTALLINE POLYMERS PHASE EMULSION, ADVANCES IN POLYMER SCIENCE,* 1996, vol. 126, 163-214
- **REVOL, J.-F.** *In vitro chiral nematic ordering of chitin crystallites*
- **MARCHESSAULT, R.H.** *Int. Journal of Biological Macromolecules,* 1993, vol. 15, 329-335
- **BELAMIE, E ; DAVIDSON, P ; GIRAUD-GUILLE, M.M.** Structure and chirality of the nematic phase in chitin suspensions. *Journal of Physical Chemistry B,* 2004, vol. 108 (39), 14991-15000
- **FAN et al.** *Carbohydrate polymers,* 2012, vol. 79, 1046-1051